# EUROPEAN PATENT APPLICATION

(11) **EP 2 202 233 A1**
(43) Date of publication of application: **30.06.2010**
(21) Application number: 08840583.2
(22) Date of filing: 17.10.2008
(51) Int. Cl.: C07D 471/04, A61K 31/437, A61P 25/22, A61P 25/24, A61P 43/00, C07F 5/02

(54) **METHOD FOR PRODUCING PYRAZOLE FUSED RING DERIVATIVE**

(30) Priority: 18.10.2007 JP 2007271068; 17.04.2008 JP 2008107868
(71) Applicant: Eisai R&D Management Co., Ltd., Tokyo 112-8088 (JP)
(72) Inventor: SATO, Keizo, Tsukuba-shi Ibaraki 300-2635 (JP); NARA, Kazumasa, Tsukuba-shi Ibaraki 300-2635 (JP); SHIMOMURA, Naoyuki, Tsukuba-shi Ibaraki 300-2635 (JP)
(74) Representative: HOFFMANN EITLE
(86) International application number: PCT/JP2008/068822
(87) International publication number: WO 2009/051210

(57) **Abstract**

Disclosed is a commercially advantageous method for producing a pyrazole fused ring derivative (such as a 7-phenylpyrazolo[1,5-a]pyridine derivative). Specifically disclosed is a method for producing a compound (I) represented by the formula (I) below or a salt thereof, which comprises a step A wherein a hydroxy group in a compound (IV) represented by the formula (IV) below is converted into a methoxy group, thereby obtaining a compound (I) or a salt thereof:

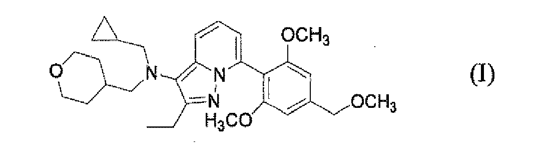

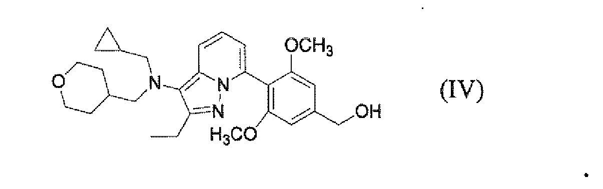

## Description

### Technical Field

The present invention relates to a method for producing pyrazole-fused ring derivatives, and the intermediates in the production method.

### Background Art

As a corticotropin-releasing-factor (CRF) preceptor antagonist, pyrazole-fused ring derivatives, for example, *N*-cyclopropylmethyl-7-(2,6-dimethoxy-4-methoxymethylphenyl) -2-ethyl-*N-*(tetrahydro-2*H*-pyran-4-ylmethyl)pyrazolo[1,5-*a*]p yridin-3-amine) (referred herein to a compound (I)), which is useful for prevention or treatment of depression, anxiety and the like, were known.

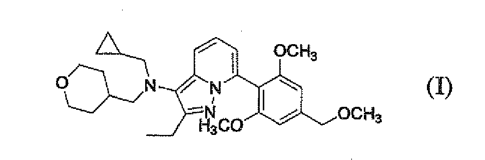

For the compound, there are reports on (1) 7-phenylpyrazolo[1,5-*a*]pyridine derivatives (see Patent Document 1); and (2) a method for producing 7-phenylpyrazolo[1,5-*a*]pyridine derivatives (see Patent Document 2).
Patent Document 1: International Publication No. 2004/037822.
Patent Document 2: International Publication No. 2005/100354.

### Disclosure of Invention

### Problems to be solved by Invention

However, the reaction step of introducing an aryl group to a pyrazole-fused ring derivative in a respective method for producing pyrazole-fused ring derivatives (7-phenylpyrazolo[1,5-*a*]pyridine derivatives and the like) described in each of the documents is not so satisfactory as an industrial production method.

An object of the present invention is to provide an industrially useful method for producing the compound (I).

Further, an object of the present invention is to provide novel raw compounds, which can be used in the method for producing the compound (I), or novel intermediates in the method.

### Means for solving the Problems

Taking the above-described things into consideration, the present inventors have intensively investigated, energetically studied, and resultantly have found following inventions:
<1> A method for producing a compound (I) represented by following formula, or a salt thereof, comprising:
   a step A of converting a hydroxyl group in a compound (IV) represented by following formula into a methoxy group, thereby to obtain the compound (I) or the salt thereof: 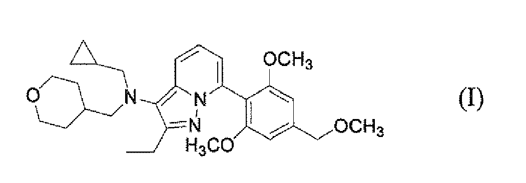 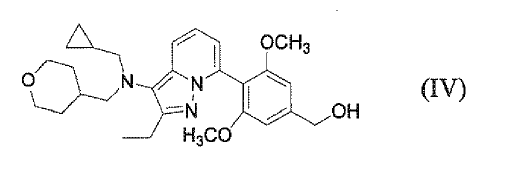
<2> In the above item <1>, the step A may be carried out by using a methylating agent.
<3> In the above item <1>, the step A may comprise a step A-1 of converting a hydroxyl group of the compound (IV) into a leaving group, and a step A-2 of substituting the leaving group with a methoxy group.
<4> In the above item <3>, the step A-1 may be carried out by using at least one selected from the group consisting of sulfonyl chloride, thionyl halide and phosphorus halide.
<5> In the above item <3>, the step A-2 may be carried out by using a combination of an alkali metal hydroxide and methanol, or a metal alkoxide.
<6> In any one of the above items <1> to <5>, the method may further comprise, before the step A, a step B of reacting a compound (II) represented by the following formula or a salt thereof with a compound (III) represented by the following formula, to obtain the compound (IV): 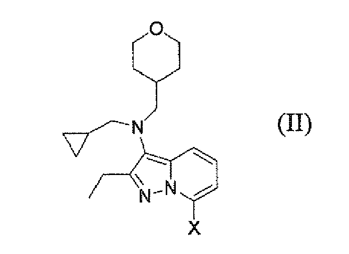 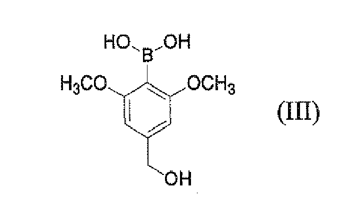 wherein X represents a halogen.
<7> In any one of the above items <1> to <5>, the method may further comprise, before the step A, a step B of reacting a compound (II) represented by the following formula or a salt thereof with a compound (III') represented by the following formula, and converting, in a case where R¹ is a protective group P¹, -OR¹ in the resulting compound into a hydroxyl group, to obtain the compound (IV): 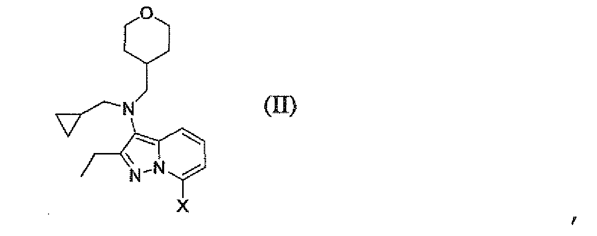 wherein X represents a halogen; 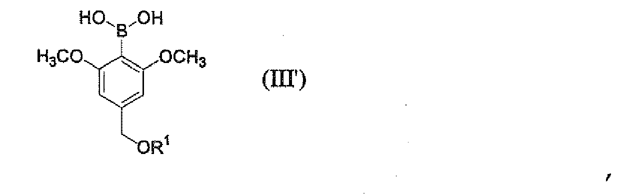 wherein R¹ represents a hydrogen atom or a protective group P¹; P¹ represents a group selected from the group consisting of a methyl group which may have substituents, an ethyl group which may have substituents, a benzyl group which may have substituents and a silyl group.
<8> In the above item <7>, the method may comprise, before the step B, a step of removing the protective group P¹ in the compound (III'') represented by the following formula, to obtain a compound (III) represented by the hollowing formula as the compound (III'): 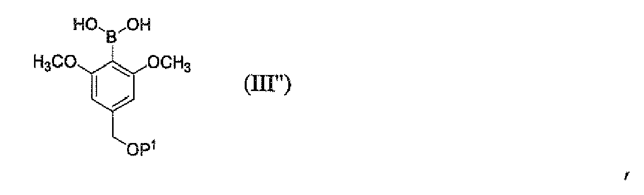 wherein the protective group P¹ has the same definition as described above; 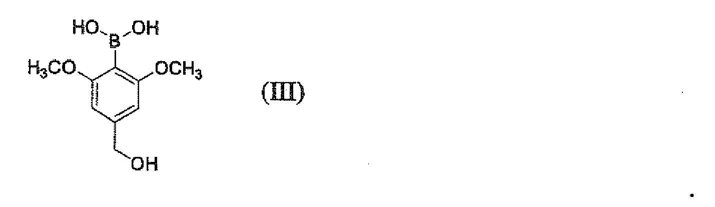
<9> A method for producing a compound (I) or a salt thereof, comprising a step of reacting a compound (II) represented by the following formula or a salt thereof, with a compound (V) represented by the following formula or a compound (VI) represented by the following formula, to obtain the compound (I) represented by the following formula or a salt thereof: 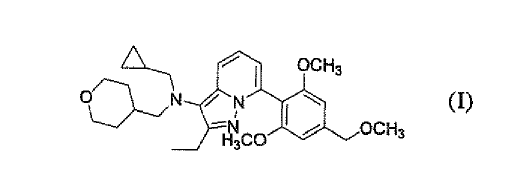 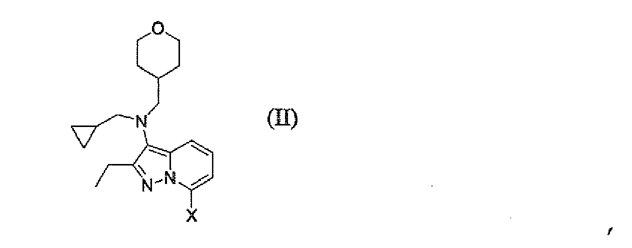 wherein X represents a halogen; 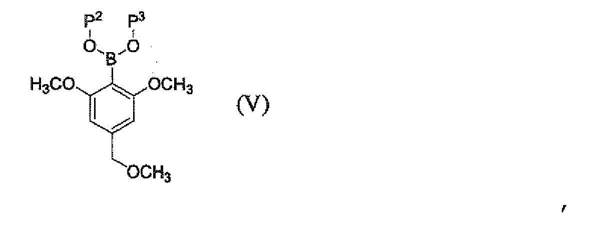 wherein P² and P³ are joined together with -O-B-O- to represent a group represented by any one of the following formulae A-1 to A-4: 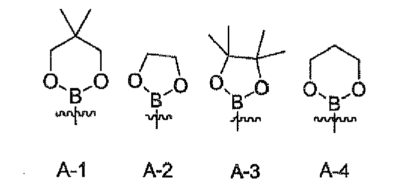 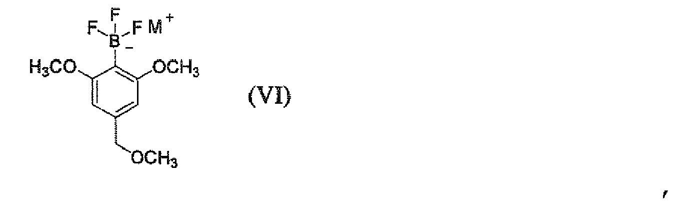 wherein M represents an alkali metal, an alkaline earth metal or an ammonium.
<10> A compound (IV') represented by the following formula or a salt thereof: 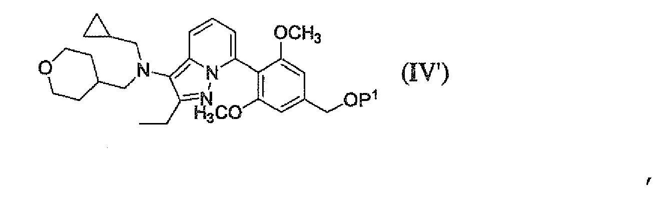 wherein P¹ represents a group selected from the group consisting of a methyl group which may have substituents, an ethyl group which may have substituents, a benzyl group which may have substituents and a silyl group.
<11> A compound (III") represented by the following formula, a compound (V) represented by the following formula, a compound (VI) represented by the following formula, or a salt thereof: 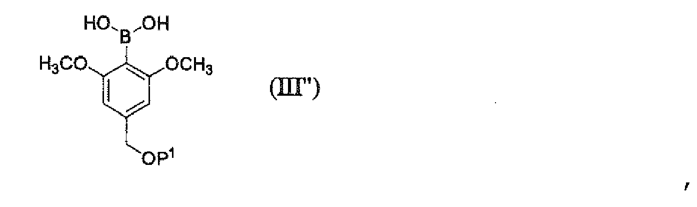
wherein P¹ represents a group selected from the group consisting of a methyl group which may have substituents, an ethyl group which may have substituents, a benzyl group which may have substituents, and a silyl group; 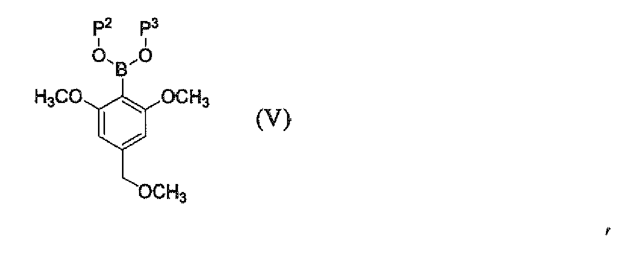 wherein P² and P³ are joined together with -O-B-O- to represent a group represented by any one of the following formulae A-1 to A-4; 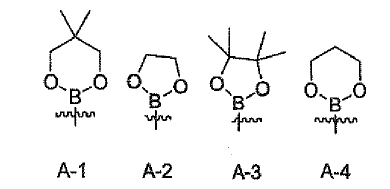 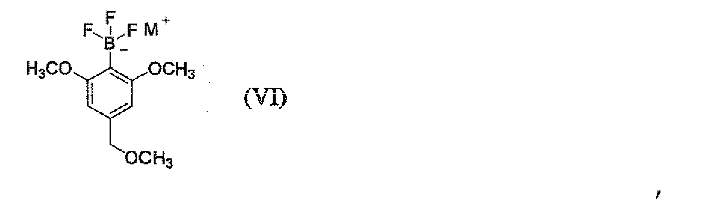 wherein M represents an alkali metal, an alkaline earth metal or an ammonium.

### Effects of Invention

The present invention can provide an industrially useful method for producing the compound (I) which is a pyrazole-fused ring derivative.

Further, the present invention can provide novel raw compounds, which can be used in the method for producing the compound (I), or novel intermediates in the method.

### Preferred Embodiments to Carry Out the Present Invention

The present invention will be described in detail hereinafter.

Several of the structural formulae given for compounds throughout the present specification will represent a specific isomer for convenience, but the invention is not limited to such specific isomers and encompasses all isomers and isomer mixtures, including geometric isomers, asymmetric carbon-derived optical isomers, stereoisomers and tautomers, implied by the structures of the compounds.

Further, the compounds of the present invention may form salts, and the compounds of the present invention may encompass anhydrates, hydrates, or solvates of the compounds or the salts. Unless stated otherwise, the compounds of the present invention may be, but are not limited to a certain crystal form, crystalline or noncrystalline.

### [Definition of X]

X represents a halogen atom. Preferably, X represents a bromine atom or an iodine atom.

### [Definition of M]

M represents an alkali metal, an alkaline earth metal or an ammonium. Specific examples of the alkali metal may include Li, Na, K and Cs. Specific examples of the alkaline earth metal may include Mg, Ca and Ba. Specific examples of the ammonium may include NH₄, NH₄ which may have substituents, and the like. However, in the compound (VI), when M is an alkaline earth metal, M corresponds to an anion in an amount of two-fold moles.

### [Definition of R¹ and P¹]

R¹ represents a hydrogen atom or a protective group P¹.

P¹ represents a group selected from the group consisting of a methyl group which may have substituents, an ethyl group which may have substituents, a benzyl group which may have substituents, and a silyl group.

Specific examples of the methyl group which may have substituents may include a methoxymethyl group, a methylthiomethyl group, a *t*-butylthiomethyl group, a (phenyldimethylsilyl)methoxymethyl group, a benzyloxymethyl group, a *p*-methoxybenzyloxymethyl group, a (4-methoxyphenoxy)methyl group, a guaiacolmethyl group, a *t*-butoxymethyl group, a 4-pentenyloxymethyl group, a siloxymethyl group, a 2-methoxyethoxymethyl group, a 2,2,2-trichloroethoxymethyl group, a bis(2-chloroethoxy)methyl group, a 2-(trimethylsilyl)ethoxymethyl group, a tetrahydropyranyl group, a 3-bromotetrahydropyranyl group, a tetrahydrothiopyranyl group, a 1-methoxycyclohexyl group, a 4-methoxytetrahydropyranyl group, a 4-methoxytetrahydrothiopyranyl group, a 4-methoxytetrahydrothiopyranyl *S*,*S*-dioxide group, a 1-[(2-chloro-4-methyl)phenyl]-4-methoxypiperidin-4-yl group, a 1,4-dioxan-2-yl group, a tetrahydrofuranyl group, a tetrahydrothiofuranyl group, a 2,3,3a,4,5,6,7,7a-octahydro-7,8,8-trimethyl-4,7-methanobenz ofuran-2-yl group, and the like.

Specific examples of the ethyl group which may have substituents may include a 1-ethoxyethyl group, a 1-propoxyethyl group, a 1-isopropoxyethyl group, a 1-(2-chloraethoxy)ethyl group, a 1-methyl-1-methoxyethyl group, a 1-methyl-1-benzyloxyethyl group, a 1-methyl-1-benzyloxy-2-fluoroethyl group, a 2,2,2-trichloroethyl group, a 2-trimethylsilylethyl group, a 2-(phenylselenyl)ethyl group, a *t*-butyl group, an allyl group, and the like.

Specific examples of the benzyl group which may have substituents may include a *p*-methoxybenzyl group, a 3,4-dimethoxybenzyl group, an o-nitrobenzyl group, a *p*-nitrobenzylgroup, a p-halobenzyl group, a 2,6-dichlorobenzyl group, a *p*-cyanobenzyl group, a *p*-phenylbenzyl group, a 2- and 4-picolyl group, a 3-methyl-2-picolyl *N*-oxide group, a diphenylmethyl group, a *p*,*p'*-dinitrobenzhydryl group, a 5-dibenzosuberyl group, a triphenylmethyl group, an *a*-naphthyldiphenylmethyl group, a *p*-methoxyphenyldiphenylmethyl group, a di(*p*-methoxyphenyl)phenylmethyl group, a tri(*p-*methoxyphenyl)methyl group, a 4-(4'-bromophenacyloxyphenyl)diphenylmethyl group, a 4,4',4"-*tris*(4,5-dichlorophthalimidophenyl)methyl group, a 4,4',4"-*tris*(levulinoyloxyphenyl)methyl group, a 4,4',4"-*tris*(benzoyloxyphenyl)methyl group, a 3-(imidazol-1-ylmethyl)bis(4',4"-dimethoxyphenyl)methyl group, a 1,1-bis(4-methoxyphenyl)-1'-pyrenylmethyl group, a 9-anthryl group, a 9-(9-phenyl)xanthenyl group, a 9-(9-phenyl-10-oxo)anthryl group, a benzisothiazolyl *S*,*S*-dioxide group, and the like.

Specific examples of the silyl group may include a trimethylsilyl group, a triethylsilyl group, a triisopropylsilyl group, a dimethylisopropylsilyl group, a diethylisopropylsilyl group, a dimethylthexylsilyl group, a *t*-butyldimethylsilyl group, a *t*-butyldiphenylsilyl group, a tribenzylsilyl group, a tri-*p*-xylylsilyl group, a triphenylsilyl group, a diphenylmethylsilyl group, a *t*-butylme-thoxyphenylsilyl group, and the like.

P¹ is preferably a methoxymethyl group, a tetrahydropyranyl group, a 1-ethoxyethyl group, a 1-propoxyethyl croup, or a 1-isopropoxyethyl group. [Definition of P² and P³]

P² and P³ are joined together with -O-B-O- to represent a group represented by any one of the following formulae A-1 to A-4.

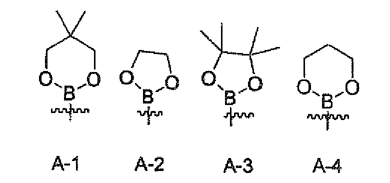

A compound or a salt thereof used herein means including the solvate thereof. The salvation is not particularly limited, as long as a solvate is formed with the compound according to the present invention or salts thereof. A solvent forms a solvate at an appropriate ratio of 0.1 to 5 molecules based on one molecule of the compound. The solvent in the solvate includes 1 to 3 solvents selected from the group consisted of dimethyl carbonate, diethyl carbonate, methyl acetate, ethyl acetate, isopropyl acetate, isopropanol, *n*-propanol and ethanol (in the case of combination of a plurality of solvents, mixtures of any ratio), and the like, and preferably solvate with dimethyl carbonate and isopropanol (mixture of any ratio), and the like.

A salt of a compound used herein may not be particularly restricted so long as it is a salt formed with the compound. Examples thereof may be salts with inorganic acids, salts with organic acids and salts with acidic amino acids, among which pharmacologically acceptable salts are preferable. The acids may form salts at appropriate ratios of 0.1-5 molecules based on one molecule of the compound.

Preferred examples of salts with inorganic acids may include salts with hydrochloricacid, hydrobromic acid, sulfuric acid, nitric acid, phosphoric acid and the like. As preferred examples of salts with organic acids, there may be salts with acetic acid, succinic acid, fumaric acid, maleic acid, tartaric acid, citric acid, lactic acid, stearic acid, benzoic acid, methanesulfonic acid, p-toluenesulfonic acid and the like.

Preferred examples of salts with acidic amino acids may include salts with aspartic acid, glutamic acid and the like.

Preferred examples of salts may include salts with hydrochloric acid, sulfuric acid, methanesulfonic acid, *p*-toluenesulfonic acid, or hydrobromic acid, more preferably salts with hydrochloric acid or p-toluenesulfonic acid.

Next, the production method according to the present invention will be described in detail.

In the various formulae shown below, X, M, R¹ and P¹ to P³ have the same definitions as described above.

### [Step 1]

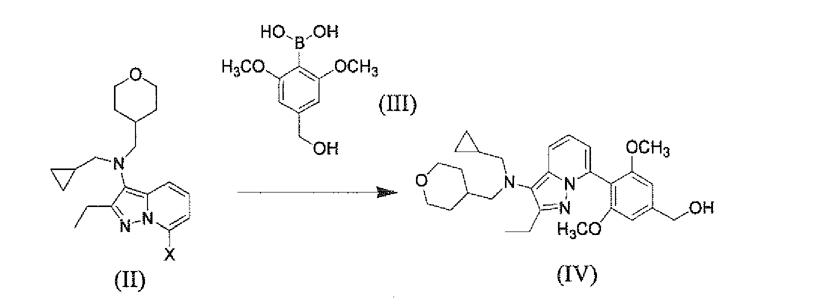

Step 1 is a step in which a compound (II) or a salt thereof is reacted with a compound (III) to obtain a compound (IV). Conditions for the reaction, treatment, purification and the like for the Step 1 will be described below, and as a specific example, the reaction can be carried out by referring to the following Example B.

The compound (II) can be produced by methods described in WO 2004/037822, WO 2005/100354 and the like.

The compound (III) is commercially available (CAS Registry NO.: 332394-37-3). The compound (III) can also be produced by, for example, removing the protective group P¹ of a compound (III"). As a specific example, the compound can be produced by referring to the following Examples A-1 to A-5.

The compound (IV), which is a result of substituting the halogen at the 7-position of the compound (II) with an aryl group derived from the compound (III), can be obtained by allowing the compound (III), a metalloaryl reagent, to react with the compound (II) using a transition metal catalyst, in the presence or absence of a base and in a solvent or without solvent. The reaction temperature is usually 0°C to 200°C.

Generally used combinations of reagent and catalyst include arylboric acid compound/palladium catalyst (Suzuki reaction; N. Miyaura, A. Suzuki, Chemical Reviews 1995, 95, 2457), aryltrialkyltin compound/palladium catalyst (Stille reaction; T.N. Mitchell, Synthesis 1992, 803), arylzinc compound/palladiumcatalyst, and aryl-Grignard compound/nickel catalyst. Specific palladium and nickel catalysts that are used, depending on the solvent used or the like, are not particularly limited as long as the catalyst does not inhibit the reaction. Suitable examples may include tetrakis(triphenylphosphine)palladium(0), palladium(II) acetate/triphenylphosphine, palladium(II) chloride, tris(dibenzylideneacetone)dipalladium(0)/tri-*tert*-butylphos phine, dichloro[1,1'-bis(diphenylphosphine)-ferrocene]palladium(0), [1,2-bis(diphenylphosphino)ethane]dichloronickel(II), [1,3-bis(diphenylphosphino)propane]dichloronickel(II), and the like.

The solvent used, depending on the reagent or the like, is not particularly limited as long as the solvent does not inhibit the reaction and dissolves the starting materials to a certain extent. Suitable examples thereof may include toluene, xylene, mesitylene, anisole, *N*,*N*-dimethylformamide, 1,2-dimethoxyethane, tetrahydrofuran, 1,4-dioxane, *n*-butanol, ethanol, methanol, 1-methyl-2-pyrrolidinone, acetone, water and the like, and these are used singly or as mixed solvents.

The base used, depending on the solvent used or the like, is not particularly limited as long as the base does not inhibit the reaction. Suitable examples may include potassium carbonate, sodium carbonate, barium hydroxide, cesium fluoride, potassium fluoride, sodium hydrogen carbonate, triethylamine and the like. In this case, it is preferable to use 1 to 2 equivalents of the metalloaryl reagent, 0.001 to 0.2 equivalents of the transition metal catalyst, and 1 to 5 equivalents of the base, based on the compound (II).

### [Step 2]

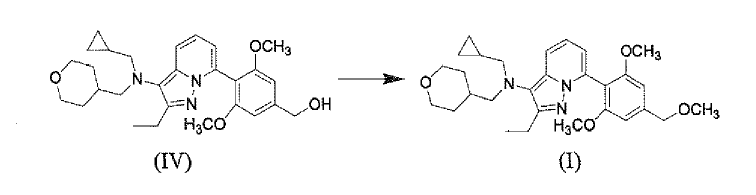

Step 2 is a step in which the hydroxyl group in the compound (IV) is converted to a methoxy group to obtain a compound (I) or a salt thereof. This step may be carried out in a single step, or may be carried out through a plurality of steps. An example of converting to a methoxy group through a plurality of steps involves conversion based on a step of converting the hydroxyl group of the compound (IV) to a leaving group, and a step of substituting the leaving group with a methoxy group. Conditions of the reaction, treatment, purification and the like for the Step 2 will be described below, as a specific example, the reaction can be carried out by referring to the following Examples D-1 to D-4.

The compound (IV) can be produced by the method of the Step 1.

In the case of carrying out the conversion into a methoxy group in a single step, this step can be carried out by using a methylating agent (dimethyl sulfate, methyl halide such as methyl iodide, or the like). This reaction is preferably carried out in the presence of a base and in a solvent. Although not particularly limited, the reaction temperature is preferably 0 to 70°C, and the reaction time is preferably 0.5 to 6 hours. Further, it is preferable to carry out the reaction in open air (preferably, free of water) or under an inert gas such as N₂.

The base used, depending on the solvent used or the like, is not particularly limited as long as the base does not inhibit the reaction. Suitable examples may include sodium hydride; metal alkoxides such as potassium *t*-butoxide; alkali metal hydroxides such as sodium hydroxide, potassium hydroxide and lithium hydroxide; and the like.

The solvent used, depending on the reagent or the like, is not particularly limited as long as the solvent does not inhibit the reaction and dissolves the starting materials to a certain extent. Suitable examples thereof may include ether type solvents such as tetrahydrofuran, 1,2-dimethoxyethane and the like; aromatic hydrocarbon type solvents such as toluene, xylene and the like; *N*,*N*-dimethylformamide, N-methylpyrrolidone, and the like. These solvents are used singly or as mixed solvents.

These reagents, bases and the like are commercially available. Further, it is preferable to use 0.5 to 5 equivalents of the reagent such as dimethyl sulfate, and 1 to 3 equivalents of the base, based on the compound (IV).

In the case of carrying out the conversion into a methoxy group in two steps, the conversion can be carried out through a step of converting the hydroxyl group of the compound (IV) to a leaving group, and a step of substituting the leaving group with a methoxy group, as described above. These steps can be carried out as a series of operations, without performing purification based on column chromatography or the like in the middle of the step, and the compound (I) can be efficiently produced.

The step of converting to a leaving group can be carried out by using at least one reagent selected from the group consisting of sulfonyl chloride (*p*-toluenesulfonyl chloride, methanesulfonyl chloride or the like), thionyl halide (thionyl chloride, thionyl bromide or the like) and phosphorus halide. This step is preferably carried out in a solvent. Although not particularly limited, the reaction temperature is preferably 0 to 120°C, and the reaction time is preferably 0.5 to 24 hours. The reaction is preferably carried out in open air (preferably, free of water) or under an inert gas such as N₂.

The step of substituting with a methoxy group can be carried out by using a combination of an alkali metal hydroxide (sodium hydroxide, potassium hydroxide, lithium hydroxide or the like) and methanol, or a metal alkoxide (a metal methoxide such as sodium methoxide, potassium methoxide or lithium methoxide, or the like). This step is preferably carried out in a solvent. Although not particularly limited, the reaction temperature is preferably 0 to 120°C, and the reaction time is preferably 0.5 to 24 hours. The reaction is preferably carried out in open air (preferably, free of water) or under an inert gas such as N₂.

The solvent used, depending on the starting raw material, reagent or the like, is not particularly limited as long as the solvent does not inhibit the reaction and dissolves the starting materials to a certain extent. Suitable examples thereof may include toluene, 1,2-dimethoxyethane, methanol and the like, and these are used singly or as mixed solvents.

These reagents and bases are commercially available. Further, it is preferable to use 1 to 3 equivalents of the reagent for converting to a leaving group, and 2 to 20 equivalents of the reagent for substituting with a methoxy group, based on the compound (IV).

After the reaction treatment as described above, a desired compound can be obtained by carrying out, if necessary, an appropriate combination of (1) a conventional extraction treatment using an organic solvent/aqueous solution, drying of the organic solvent using magnesium sulfate or the like, and removal by evaporation of the solvent of the solution; (2) purification by column chromatography or purification by recrystallization from an appropriate solvent; and (3) filtration of a product generated by adding an organic solvent and water (a mixture of ethyl acetate and water, or the like), or the like.

Examples of the organic solvent that can be used in the extraction treatment using an organic solvent/aqueous solution, may include ester type solvents such as ethyl acetate and isopropyl acetate; ether type solvents such as *t*-butyl methyl ether; hydrocarbon type solvents such as toluene; and mixed solvents thereof. The extraction treatment can be carried out a single time or several times. As a work-up treatment, a method of adding water to the reaction mixture and then filtering the mixture, can also be used. Various salts, inorganic base and organic base compounds can be dissolved and used as the aqueous solution. When an acidic aqueous solution of hydrochloric acid or the like is used, the target product may be extracted into the aqueous layer.

### [Step 3]

Alternatively, a compound (III") represented by the following formula can be used in place of the compound (III) of the Step 1.

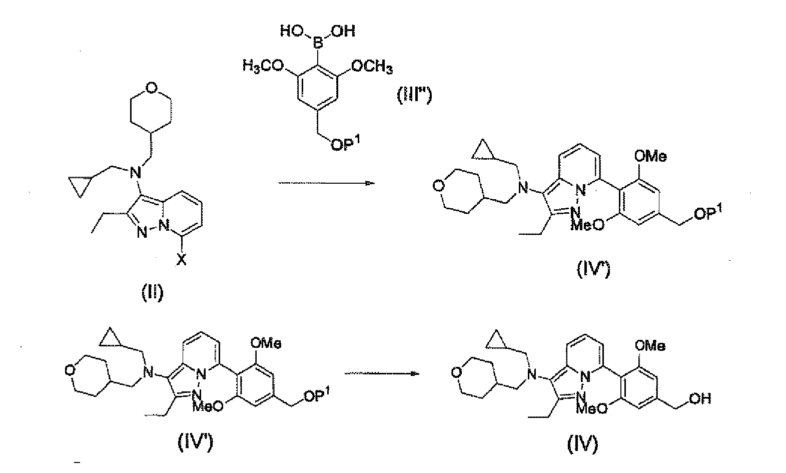

Step 3 is a step in which the compound (II) or a salt thereof is reacted with a compound (III'), and -OP¹ in the resulting compound (IV') is converted to a hydroxyl group, to obtain the compound (IV). By using this compound (III"), the compound (I) can be provided via the compound (IV'), which is a new intermediate. That is, the compound (I) can be provided by removing the protective group P¹ in the compound (IV') to obtain the compound (IV) having a -OH group, and then treating the compound (IV) by the method described above.

In the Step 3, conditions for the reaction, treatment, purification and the like concerning the coupling step of the compound (II) and compound (III") can be set up in a manner similar to the Step 1. As a specific example, the reaction can be carried out by referring to the following Example C-2.

Although dependent on the protective group P¹, the compound (III") can be obtained by the following scheme when, for example, a 1-ethoxyethyl group is used as P¹. In addition, the compound can be produced according to this scheme even with other P¹'s. As a specific example, the compound (III") can be produced by referring to Examples A-1 to A-3 and C-1.

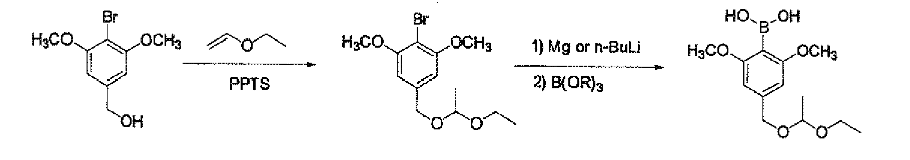

The first step of the above scheme is a step for the protection of a hydroxyl group. This step is dependent on the protective group P¹ used, but with regard to a 1-ethoxyethyl group, it is preferable to allow ethyl vinyl ether to react at 0 to 30°C in the presence of an acid catalyst (pyridinium *p*-toluenesulfonate, p-toluenesulfonic acid monohydrate, or the like). As the solvent, an ether type solvent (*tert*-butyl methyl ether, cyclopentyl methyl ether, tetrahydrofuran, or the like) can be used.

The second step of the scheme is a step of converting -Br into -B(OH)₂. This step is dependent on the protective group P¹ used, but it is preferable that a Grignard reagent is generated using magnesium or -Br is lithiated using alkyllithium (*n*-butyllithium or the like), subsequently the product is treated with trialkyl borate (trimethyl borate, triisopropyl borate or the like) to obtain a boric acid ester, and the ester is hydrolyzed under weak acidic conditions such as an aqueous solution of ammonium chloride. The reaction temperature is preferably 30 to 120°C for the preparation of Grignard reagent, -80 to -50°C for lithiation, -80 to 30°C for boric acid ester formation, and -20 to 30°C for hydrolysis. As the solvent, an ether type solvent such as tetrahydrofuran can be used.

In the Step 3, conditions for the reaction, treatment, purification and the like concerning the step for deprotection of the compound (IV') will be described below, as a specific example, the reaction can be carried out by referring to the following Example C-3.

Although dependent on the protective group P¹ used, this step can be easily carried out at room temperature under acidic conditions when an alkoxymethyl group, a tetrahydropyranyl group or a 1-alkoxyethyl group is used as P¹.

### [Step 4]

Alternatively, a compound (V) or a compound (VI) can be used in substitution of the compound (III) of the Step 1.

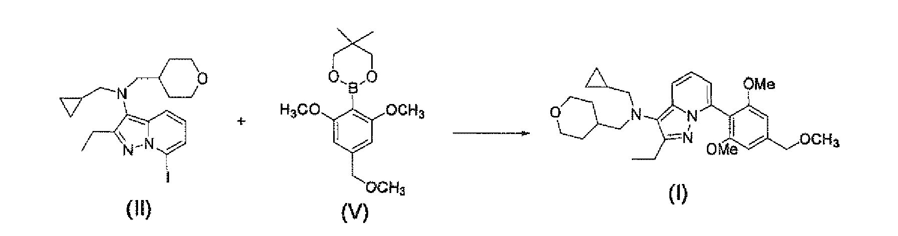

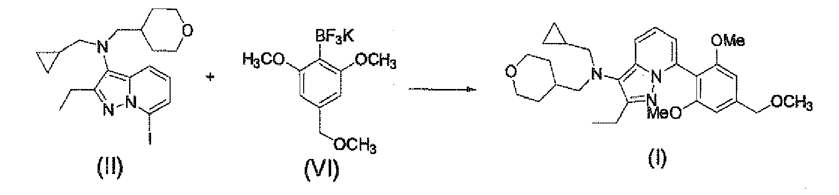

Step 4 is a step in which the compound (II) or a salt thereof is reacted with the compound (V) or compound (VI) to obtain the compound (I) or a salt thereof. When the compound (V) or compound (VI) is reacted with the compound (II), the compound (I) can be obtained directly without going through the compound (IV).

The step of reacting the compound (II) or a salt thereof with the compound (V) or the compound (VI) is dependent on the P² and P³ used or on the M used, but the step can be carried out under the same conditions as those of the Step 1. As a specific example, the compound (I) can be produced by referring to Example E-2 in the case of using the compound (V), and by referring to Example F-2 in the case of using the compound (VI).

Although dependent on the protective groups P² and P³ used, the compound (V) can be obtained by the following scheme when, for example, the formula A-1 described above is used as P² and P³. In addition, the compound can be produced according to this scheme even with other P²'s and P³'s. As a specific example, the compound (V) can be produced by referring to Examples E-1-1 and E-1-2.

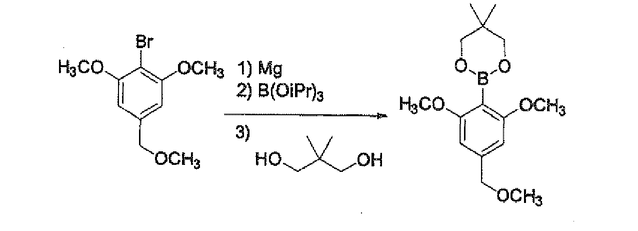

In the above scheme, the step can be carried out such that a Grignard reagent is generated using magnesium, subsequently trialkyl borate (trimethyl borate, triisopropyl borate or the like) is added to the Grignard reagent to obtain a boric acid ester, and the ester can be converted to a cyclic ester by using 2,2-dimethyl-1,3-propanediol. The reaction temperature is preferably 30 to 120°C for the preparation of Grignard reagent, -80 to 30°C for the boric acid ester formation, and 0 to 30°C for the conversion into a cyclic ester. As the solvent, an ether type solvent such as tetrahydrofuran can be used.

The compound (VI), depending on the M used, for example, when potassium (K) is used as M, can be obtained by the following scheme. In addition, the compound can be produced according to this scheme even with other M's. As a specific example, the compound (VI) can be produced by referring to Example F-1.

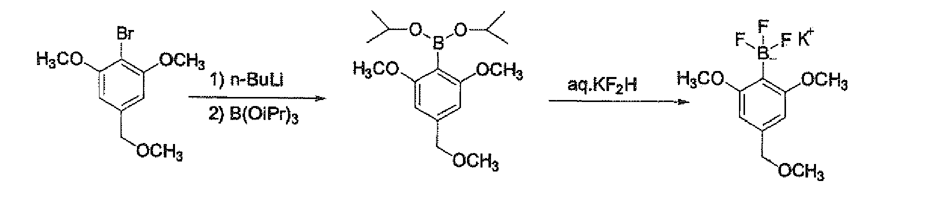

The first step of the scheme is a step of converting -Br into -B(OⁱPr)₂. This step is preferably carried out such that lithiation and dropwise addition of the boric acid ester are carried out at -80 to 50°C, and subsequently the temperature is increased to room temperature.

The second step of the scheme is a step of converting -B(OⁱPr)₂ into -BF₃K. This step is preferably carried out such that an aqueous solution of potassium hydrogen fluoride is added dropwise at 5°C or below, and then the reaction is carried out at room temperature.

### EXAMPLES:

The present invention will be described specifically and in more detail by way of the following Examples, but the present invention is not intended to be limited thereto.

### Example A. Synthesis of [4-(hydroxymethyl)-2,6-dimethoxyphenyl]boronic acid

### Example A-1:

### Synthesis of [4-(hydroxymethyl)-2,6-dimethoxyphenyl]boronic acid (1)

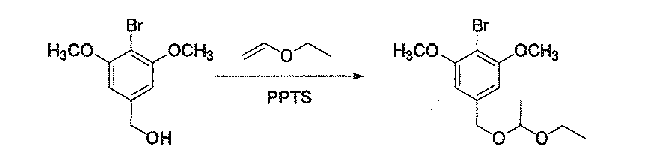

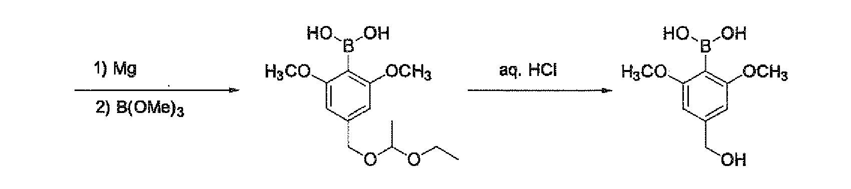

### Synthesis of

### 2-bromo-5-[(1-ethoxyethoxy)methyl]-1,3-ctimethoxybenzene

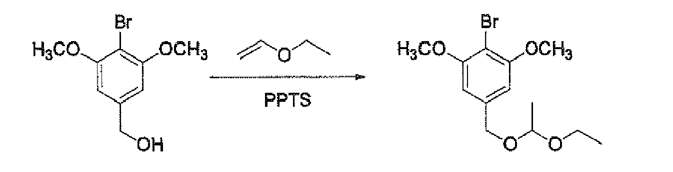

*t*-Butyl methyl ether (100 mL), ethyl vinyl ether (7.8 mL) and pyridinium *p*-toluenesulfonate (1.04 g) were sequentially added to (4-bromo-3,5-dimethoxy) phenylmethanol (10.00 g), and the mixture was stirred for 1.5 hours at room temperature. Tetrahydrofuran (15 mL) was added to the reaction solution, and the mixture was further stirred for about 15 hours. Subsequently, the reaction liquid was transferred into a separatory funnel and was washed sequentially with a 5% aqueous solution of sodium hydrogen carbonate and water. The organic layer was dried over anhydrous sodium sulfate, the resultant was filtered, and then the solvent was distilled off under reduced pressure, thereby to obtain 12. 34 g (yield 95.4%) of the title compound as a pale yellow oily matter.
¹H-NMR (400MHz, CDCl₃) δ: 1.23 (t, *J*=7.2Hz, 3H), 1.38 (d, *J*=5.2Hz, 3H), 3.52-3.58 (m, 1H), 3.64-3.72 (m, 1H), 3.90 (s, 6H), 4.49 (d, *J*=12.4Hz, 1H), 4.62 (d, *J*=12.4Hz, 1H), 4.82 (q, *J*=5.2Hz, 1H), 6.58 (s, 2H).

### Synthesis of {4-[(1-ethoxyethoxy)methyl]-2,6-dimethoxyphenyl}boronic acid

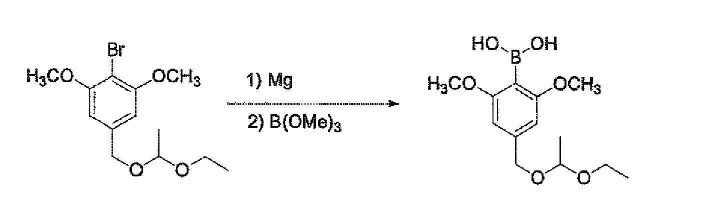

Magnesium (984 mg), tetrahydrofuran (24.6 mL) and iodine (catalytic amount) were mixed. To this mixture, about a one-third amount of a solution of 2-bromo-5-[(1-ethoxyethoxy)methyl]-1,3-dimethoxybenzene (12.29 g) in tetrahydrofuran (12.3 mL) was added, and the mixture was heated to 70°C. After it was confirmed that the color of the solution turned clear and colorless, and that the solution began to generate bubbles, the remaining portion (2/3) of the solution of 2-bromo-5-[(1-ethoxyethoxy)methyl]-1,3-dimethoxybenzene in tetrahydrofuran was injected therein over 12 minutes. The reaction mixture was stirred for about 1.5 hours at 70°C, was subsequently cooled to room temperature, and was diluted with tetrahydrofuran (36.9 mL). The reaction liquid was cooled in an ice salt bath, and trimethyl borate (8.6 mL) was added dropwise thereto over 9 minutes at -14. 9 to -8.0°C. The reaction mixture was stirred for about 1.5 hours at the same temperature, a 10% aqueous solution of ammonium chloride (98 mL) was added thereto, and the mixture was returned to room temperature. Ethyl acetate (123 mL) was added to the reaction mixture, and partition was performed (aqueous layer (1)). The organic layer was washed with water (organic layer (1), aqueous layer (2)), and the target product was extracted again from the aqueous layer (1) with ethyl acetate (61 mL). The target product was re-extracted also from the aqueous layer (2) in the same organic layer (organic layer (2)). The organic layer (1) and the organic layer (2) were combined, and the solvent was distilled off under reduced pressure. The concentration residue was azeotropically boiled with toluene (two times), to obtain 10.46 g (yield 95.6%) of the title compound as a pale orange oily matter.
¹H-NMR (400MHz, DMSO-*d*₆) δ: 1.13 (t, *J*=7.2Hz, 3H), 1.26 (d, *J*=5.2Hz, 3H), 3.43-3.49 (m, 1H), 3.57-3.63 (m, 1H), 3.69 (s, 6H), 4.44 (d, *J*=12.4Hz, 1H), 4.54 (d, *J*=12.4Hz, 1H), 4.76 (q, *J*=5.2Hz, 1H), 6.52 (s, 2H), 7.79 (s, 2H).

### Synthesis of [4-(hydroxymethyl)-2,6-dimethoxyphenyl]boronic acid

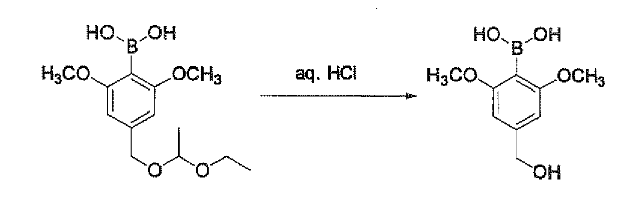

Toluene (31.3 mL)), isopropyl acetate (10.4mL)) and water (14.1 mL) were added to {4-[(1-ethoxyethoxy)methyl]-2,6-dimethoxyphenyl}boronic acid (10.44 g), and the mixture was stirred. 1 N hydrochloric acid (1.57 mL) was added to this mixture, and the mixture was stirred for about 5 hours at room temperature. The reaction mixture was cooled in an ice water bath, and precipitated crystals were collected by filtration. The crystals were washed sequentially with toluene (10.4 mL)) and water (5.2 mL), and were dried under reduced pressure at 40°C, to obtain 5.63 g (yield 72.4%) of the title compound.
¹H-NMR (400MHz, DMSO-*d*₆) δ: 3.68 (s, 6H), 4.46 (d, *J*=5.6Hz, 2H), 5.16 (t, *J*=5.6Hz, 1H), 6.52 (s, 2H), 7.75 (s, 2H).

### Example A-2:

Synthesis of [4-(hydroxymethyl)-2,6-dimethoxyphenyl]boronic acid (2)

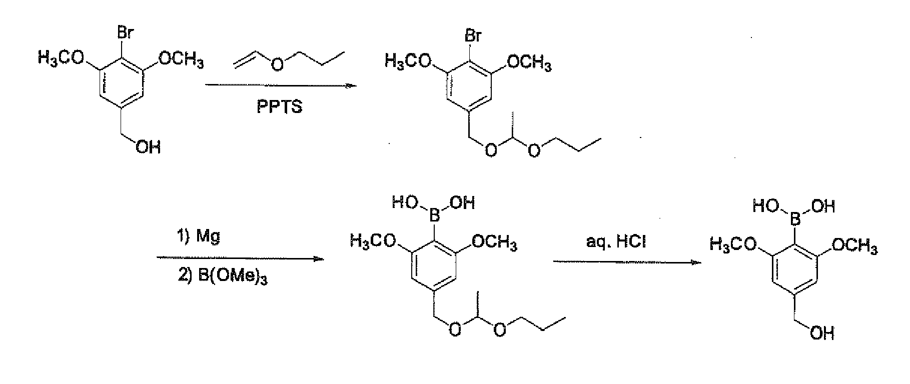

### Synthesis of 2-bromo-1,3-dimethoxy-5-[(1-propoxyethoxy)methyl]benzene

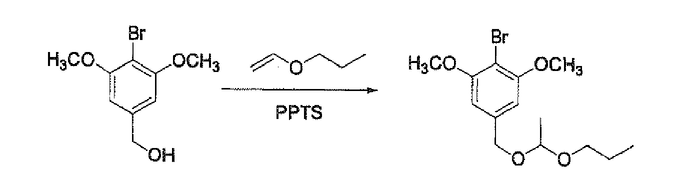

Cyclopentyl methyl ether (50 mL), tetrahydrofuran (5 mL), propyl vinyl ether (4.55mL) and pyridinium *p*-toluenesulfonate (517 mg) were sequentially added to (4-bromo-3,5-dimethoxyphenyl)methanol (5.00 g), and the mixture was stirred for about 4 hours at room temperature. The reaction liquid was washed sequentially with a 5% aqueous solution of sodium hydrogen carbonate and water. The solvent was distilled off under reduced pressure, and the residue was azeotropically boiled with cyclopentyl methyl ether, to obtain 7.68 g (content 6.76g, yield: quantitative) of the title compound as a pale orange oily matter.
¹H-NMR (400MHz, CDCl₃) δ: 0.96 (t, *J*=7.2Hz, 3H), 1.38 (d, *J*=5.2Hz, 3H), 1.58-1.65 (m, 2H), 3.43-3.47 (m, 1H), 3.54-3.58 (m, 1H), 3.90 (s, 6H), 4.50 (d, *J*=12.4Hz, 1H), 4.63 (d, *J*=12.4Hz, 1H), 4.82 (q, *J*=5.2Hz, 1H), 6.58 (s, 2H).

### Synthesis of {2,6-dimethoxy-4-[(1-propoxyethoxy)methyl]phenyl}boronic acid

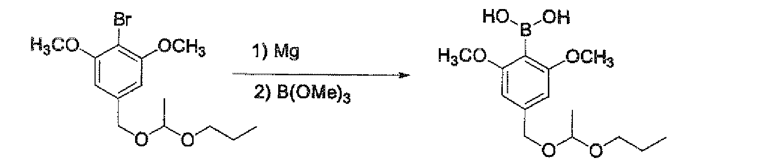

Magnesium (339 mg), tetrahydrofuran (10 mL) and iodine (catalytic amount) were mixed. To this mixture, about a one-third amount of a solution of 2-bromo-1,3-dimethoxy-5-[(1-propoxyethoxy)methyl]benzene (5.00 g) in tetrahydrofuran (5.0 mL) was added, and the mixture was heated to 70°C. After it was confirmed that the color of the solution turned clear and colorless, and that the solution began to generate bubbles, the remaining portion (2/3) of the solution of 2-bromo-1,3-dimethoxy-5-[(1-propoxyethoxy)methyl]benzene in tetrahydrofuran was injected therein over 9 minutes. The reaction mixture was stirred for about 1.5 hours at 70°C, was subsequently cooled to room temperature, and was diluted with tetrahydrofuran (15 mL)). The reaction liquid was cooled in an ice salt bath, and trimethyl borate (3.0 mL) was added dropwise thereto over 6 minutes at -10. 3 to -5.4°C. The reaction mixture was stirred for about 1.5 hours at the same temperature, and then a 10% aqueous solution of ammonium chloride (40 mL) was added thereto. The mixture was returned to room temperature. Isopropyl acetate (50 mL) was added to the reaction mixture, partition was performed, and the organic layer was washed with water. The solvent was distilled off under reduced pressure, and the concentration residue was azeotropically boiled with toluene, to obtain 3.90 g (content 2.70 g, yield 68.7%) of the title compound as an orange-colored oily matter.
¹H-NMR (400MHz, DMSO-*d*₆) δ: 0.89 (t, *J*=7.2Hz, 3H), 1.26 (d, *J*=5.6Hz, 3H), 1.50-1.56 (m, 2H), 3.35-3.41 (m, 1H), 3.49-3.55 (m, 1H), 3.69 (s, 6H), 4.44 (d, *J*=12.4Hz, 1H), 4.54 (d, *J*=12.4Hz, 1H), 4.76 (q, *J*=5.2Hz, 1H), 6.52 (s, 2H), 7.80 (s, 2H).

### Synthesis of [4-(hydroxymethyl)-2,6-dimethoxyphenyl]boronic acid

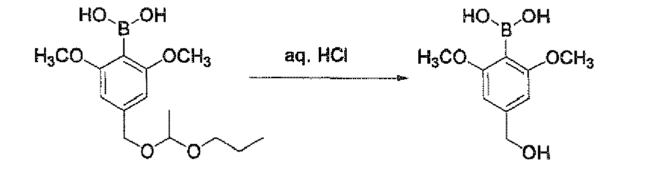

Toluene (6.0 mL), isopropyl acetate (2.0 mL) and water (2.7 mL) were added to {2,6-dimethoxy-4-[(1-propoxyethoxy)methyl]phenyl}boronic acid (2.01 g, content 1.39 g), and the mixture was stirred. 1 N hydrochloric acid (0.30 mL) was added to this mixture, and the mixture was stirred for about 20 hours at room temperature. The reaction mixture was cooled in an ice water bath, and precipitated crystals were collected by filtration. The crystals were washed sequentially with toluene (4 mL) and water (2 mL), and were dried under reduced pressure at 40°C, to obtain 835 mg (yield 84.7%) of the title compound.
The ¹H-NMR data coincided with the data of the title compound obtained in Example A-1.

### Example A-3:

### synthesis of [4-(hydroxymethyl)-2,6-dimethoxyphenyl]boronic acid (3)

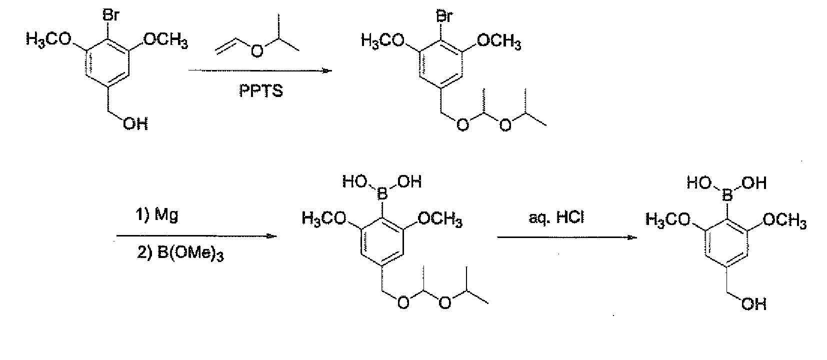

### Synthesis of 2-bromo-5-[(1-isopropoxyethoxy)methyl]-1,3-dimethoxybenzene

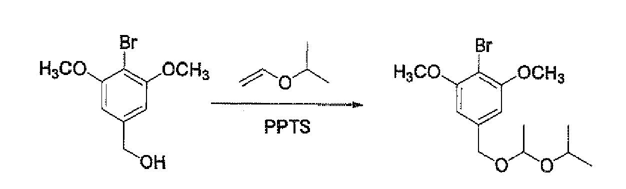

Cyclopentyl methyl ether (100 mL), tetrahydrofuran (10 mL), isopropyl vinyl ether (9.3 mL), and pyridinium *p*-toluenesulfonate (1.00 g) were sequentially added to (4-bromo-3,5-dimethoxyphenyl)methanol (10.02 g), and the mixture was stirred for about 2.5 hours at room temperature. The reaction liquid was filtered, and the filtrate was washed sequentially with a 5% aqueous solution of sodium hydrogen carbonate and water. The solvent was distilled off under reduced pressure, and the residue was azeotropically boiled with cyclopentyl methyl ether, to obtain 15.75 g (content 13.47 g, yield 99.6%) of the title compound as a pale orange oily matter. ¹H-NMR (400MHz, CDCl₃) δ: 1.18 (d, *J*=6.4Hz, 3H), 1.23 (d, *J*=6.0Hz, 3H), 1.38 (d, *J*= 5.2Hz, 3H), 3.88-3.94 (m, 1H), 3.90 (s, 6H), 4.49 (d, *J*=12.0Hz, 1H) , 4.61 (d, *J*=12.4Hz, 1H), 4.87 (q, *J=*5.2Hz, 1H), 6.58 (s, 2H).

### Synthesis of {4-[(1-isopropoxyethoxy)methyl]-2,6-dimethoxyphenyl}boronic acid

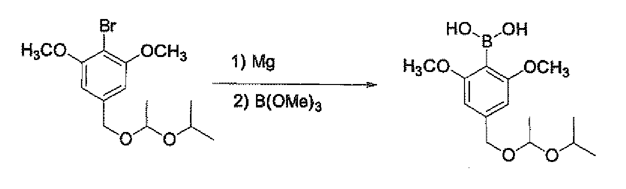

Magnesium (1.42g), tetrahydrofuran (18.6 mL) and iodine (71 mg) were mixed. To this mixture, about a one-tenth amount of a solution of 2-bromo-5-[(1-isopropoxyethoxy)methyl]-1,3-dimethoxybenzene (22.17g,content 18.58 g) intetrahydrofuran (18. 6mL) was added, and the mixture was heated to 60°C. After it was confirmed that the color of the solution turned clear and colorless, and that the solution began to generate bubbles, the remaining portion (9/10) of the solution of 2-bromo-5-[(1-isopropoxyethoxy)mathyl]-1,3-dimethoxybanzene in tetrahydrofuran was added dropwise thereto over 35 minutes. The reaction mixture was stirred for 50 minutes at 60°C and was subsequently cooled to room temperature, to prepare a solution of a Grignard reagent in tetrahydrofuran. Tetrahydrofuran (74.3 mL) was added to trimethyl borate (12.4 mL). This mixture was cooled to 0°C, and the above solution of Grignard reagent in tetrahydrofuran was added dropwise thereto over one hour. The mixture was stirred for about one hour at the same temperature, subsequently a 15% aqueous solution of ammonium chloride (149 mL) and isopropyl acetate (74 mL) were added thereto, and the mixture was returned to room temperature. The reaction mixture was filtered, and isopropyl acetate (75 mL) was added to the filtrate to perform partition. The organic layer was washed with 5% brine. The solvent was distilled off under reduced pressure, to obtain 16.53 g (content 13.57 g, yield 81.6%) of the title compound as a pale yellow oily matter. ¹H-NMR (400MHz, CDCl₃) δ: 1.18 (d, *J*=6.0Hz, 3H), 1.22 (d, *J*=6.4Hz, 3H), 1.39 (d, *J*=4.8Hz, 3H), 3.89-3.95 (m, 1H), 3.92 (s, 6H), 4.53 (d, *J*=12.8Hz, 1H), 4. 65 (d, *J*=12.8Hz, 1H), 4.89 (q, *J*=5.2Hz, 1H), 6.64 (s, 2H), 7.18 (s, 2H).

### Synthesis of [4-(hydroxymethyl)-2,6-dimethoxyphenyl]boronic acid

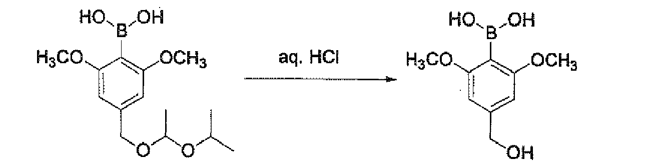

Toluene (53 mL), isopropyl acetate (18 mL) and water (24 mL) were added to (4-[(1-isopropoxyethoxy)methyl]-2,6-dimethoxyphenyl}boronic acid (17.59 g), and the mixture was stirred. I N hydrochloric acid (2.66 mL) was added to this mixture, and the mixture was stirred for about 4.5 hours at room temperature. The reaction mixture was cooled in an ice water bath, and precipitated crystals were collected by filtration. The crystals were washed sequentially with toluene (36 mL)), water (18 mL) and toluene (18 mL), and were dried under reduced pressure at 40°C, to obtain 12.02 g (yield 95.1%) of the title compound.
The ¹H-NMR data coincided with the data of the title compound obtained in Example A-1.

### Example A-4:

### Synthesis of [4-(hydroxymethyl)-2,6-dimethoxyphenyl]boronic acid (4)

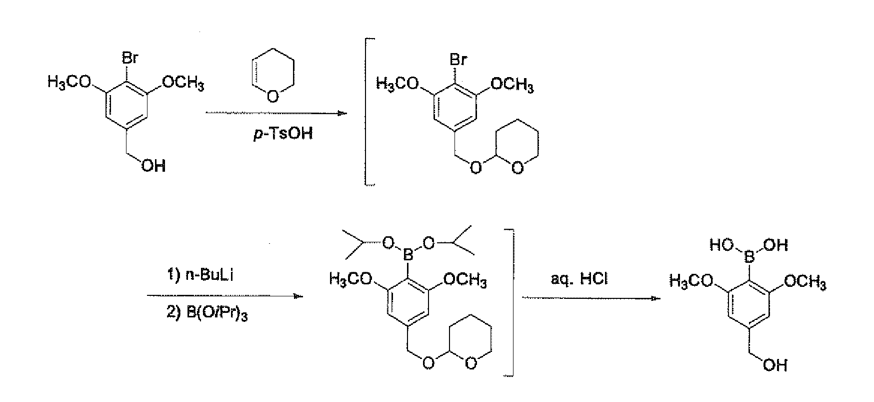

(4-Bromo-3,5-dimethoxyphenyl)methanol (2.11 g) was dissolved in tetrahydrofuran (8.3 mL), and subsequently 3,4-dihydro-2H-pyran (1.56 mL) and p-toluenesulfonic acid monohydrate (0.16 g) were added thereto. The mixture was stirred for 13.3 hours at room temperature under a nitrogen atmosphere. Tetrahydrofuran (5 mL) was added thereto, and then a 2.77 M *n*-butyllithium-hexane solution (3.3 mL) was added dropwise thereto at an internal temperature of -76.7 to -61.3°C. Three minutes after the dropwise addition, 5 mL of tetrahydrofuran was further added, and the mixture was stirred for 53 minutes in a dry ice-acetone bath. Triisopropyl borate (2.4 mL) was added dropwise thereto at an internal temperature of -76.4 to -68.7°C, and the mixture was stirred for 30 minutes at the same temperature, and then stirred for one hour at room temperature. 10 mL of 1 N hydrochloric acid was added thereto, and the mixture was stirred for 2.5 hours at room temperature. 5 N hydrochloric acid (6 mL) was added thereto, and the mixture was stirred for 2. 7 hours at the same temperature. The reaction system was left to stand still, and the lower layer was obtained by partition. A 2 N aqueous solution of sodium hydroxide was added to the lower layer to adjust to pH 7 to 8. The upper layer was extracted two times with a 2 N aqueous solution of sodium hydroxide (5 mL each), and the extracts were combined with this liquid. The obtained alkaline extracted layer was washed with *t*-butyl methyl ether (20 mL), and then was adjusted to pH = 2 to 3 with 5 N hydrochloric acid. The extracted layer was subjected to extraction four times with ethyl acetate (20 mL each). The combined ethyl acetate extracted layer was washed with 10 mL of saturated brine, and was dried over anhydrous magnesium sulfate. The residue was dried under reduced pressure at 45°C, and was dried in a vacuum at room temperature, to obtain 909 mg (yield 50.2%) of the target product.
¹H-NMR (CDCl₃): δ: 3.92 (s, 6H), 4.73 (s, 2H), 6. 61 (dd, *J*= 6.8, 1.2 Hz, 1H), 6.65 (s, 2H), 7.19 (s, 2H).

### Example A-5:

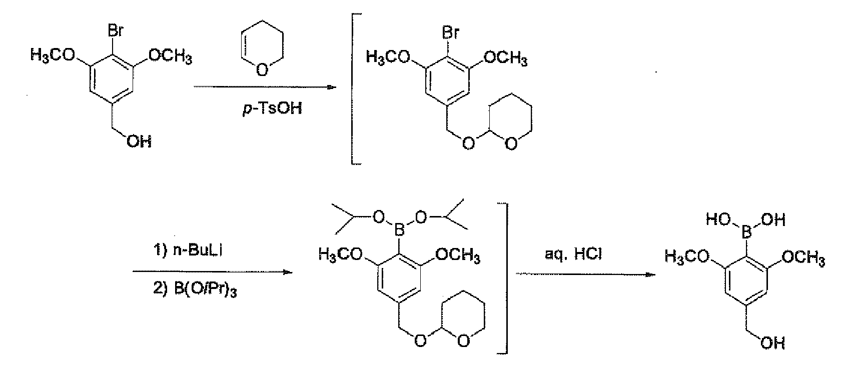

### Synthesis of [4-(hydroxymethyl)-2,6-dimethoxyphenyl]boronic acid (5)

*p*-Toluenesufonic acid monohydrate (2.3 g) and tetrahydrofuran (260 mL) were added to (4-bromo-3,5-dimethoxyphenyl)methanol (30 g) to dissolve therein, subsequently 3,4-dihydro-2*H*-pyran (12.2 mL) was added thereto, and the mixture was stirred for 17.1 hours at room temperature under a nitrogen atmosphere. 3,4-Dihydro-2H-pyran (3.8 mL) was added thereto, and the mixture was further stirred for one hour at room temperature. Tetrahydrofuran (20 mL) was added thereto. A 2. 77 M *n*-butyllithium-hexane solution (57 mL) was added dropwise thereto over 28 minutes at an internal temperature of -76.5 to -63.0°C. In the middle of this step, at the time point where 2/3 of the total amount had been added dropwise, tetrahydrofuran (40 mL) was added. Triisopropyl borate (42 mL) was added dropwise thereto at an internal temperature of -75.6 to -65.0°C, and the mixture was stirred for one hour at an internal temperature of -75°C. The mixture was stirred for 28 minutes at room temperature, subsequently 5 N hydrochloric acid (60 mL) was added thereto, and the mixture was stirred for 1. 5 hours at room temperature. Thereafter, water (15 mL) was added, and the mixture was stirred for 52 minutes. 5 N hydrochloric acid (15 mL) was further added, and the mixture was stirred for 1.1 hours. The reaction system was left to stand still, and the lower layer was obtained by partition. The upper layer was extracted with a 5 N aqueous solution of sodium hydroxide (60 mL), and the extract was combined with the lower layer previously obtained. A 5 N aqueous solution of sodium hydroxide (40 mL) was added to the resulting alkaline extract (pH 8 to 9) to adjust to pH 14, and the mixture was washed with toluene (50 mL). 5 N hydrochloric acid (50 mL) was added thereto to adjust to pH = 0 to 1, and the mixture was stirred for 45 minutes under ice cooling. Precipitates were collected by filtration and were washed with 30 mL of cold water. The precipitates were dried under reduced pressure for 10 minutes at 45°C, and subsequently were dried under reduced pressure overnight at room temperature, to obtain 11.16 g of the target product (yield 43.4%).

### Example B:

### Synthesis of (4-{3-[(cyclopropylmethyl)(tetrahydro-2H-pyran-4-ylmethyl)a mino]-2-ethylpyrazolo[1,5-a]pyridin-7-yl)-3,5-dimethoxyphen yl)methanol

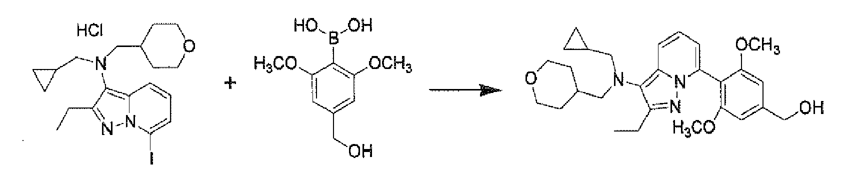

A mixture of *N*-cyclopropylmethyl-2-ethyl-7-iodo-*N-*(tetrahydro-2*H*-pyran-4-ylmethyl)pyrazolo[1,5-*a*]pyridin-3-am ine hydrochloride (10.55 g), [4-(hydroxymethyl)-2,6-dimethoxyphenyl]boronic acid (8.468g), palladium acetate (0.235 g), triphenylphosphine (1.107 g), potassium carbonate (11.62 g), 1,2-dimethoxyethane (267 mL) and water (133 mL) was heated under stirring for 11 hours and 36 minutes at 90 to 94°C under a nitrogen atmosphere. After the mixture was cooled, toluene (100 mL) was added thereto, and the aqueous layer was disposed. The organic layer was extracted with 2 N hydrochloric acid (100 mL and 33 mL). The hydrochloric acid extracted layers were combined, and toluene (150 mL) and a 5 N aqueous solution of sodium hydroxide (120 mL) were added thereto to partition the aqueous layer. Thus, the upper layer was obtained. The upper layer was washed three times with an aqueous solution of ethylenediamine (9 mL of ethylenediamine + 100 mL of water), and subsequently twice with 100 mL of water. The upper layer was concentrated under reduced pressure at 50°C. The resultant was dried under reduced pressure overnight at room temperature, to obtain 9.518 g of the target product as a yellowish white solid (quantified yield 89.9%). ¹H-NMR (CDCl₃): δ: -0.00-0.06 (m, 2H), 0.36-0.40 (m, 2H), 0.81-0.91 (m, 1H), 1.25 (t, *J*=7.6 Hz, 3H), 1.19-1.36 (m, 2H), 1.58-1.67 (m, 1H), 1.79 (br d, *J*= 11.2, 1.6 Hz, 2H), 2.80 (q, *J*=7.6 Hz, 2H), 2.90 (d, *J*=7.2 Hz, 2H), 3. 07 (d, *J* = 7.2 Hz, 2H), 3.34 (ddd, *J* = 11.6, 11.6, 2Hz, 2H), 3.74 (s, 6H), 3.96 (br dd, *J* = 11.6, 2.8 Hz, 2H), 4.65 (d, *J* = 4.4 Hz, 2H), 6.62 (dd, *J* = 6.8, 0.8 Hz, 1H), 6.69 (s, 2H), 7. 04 (dd, *J* = 8.8, 6.8 Hz, 1H), 7.48 (dd, *J* = 8.8, 0.8 Hz, 1H).

### Example C. Synthesis of {2,6-dimethoxy-4-[(tetrahydro-2H-pyran-2-yloxy)methyl]pheny 1}boronic acid, and (4-{3-[(cyclopropylmethyl)(tetrahydro-2H-pyran-4-ylmethyl)a mino]-2-ethylpyrazolo[1,5-a]pyridin-7-yl}-3,5-dimethoxyphen yl) methanol

### Example C-1: Synthesis of {2,6-dimethoxy-4-[(tetrahydro-2H-pyran-2-yloxy)methyl]phenyl}boronic acid

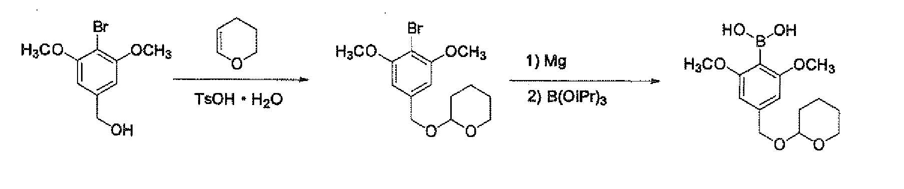

### Synthesis of 2-[(4-bromo-3,5-dimethoxybezyl)oxyl]tetrahydro-2H-pyran

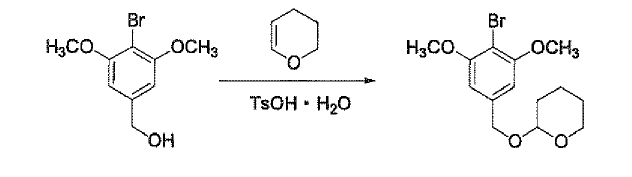

Tetrahydrofuran (25 mL) was added to (4-bromo-3,5-dimethoxyphenyl)methanol (10.03 g), and a solution of 3,4-dihydro-2H-pyran (6.82 g) in tetrahydrofuran (25 mL) was added thereto. This mixture was cooled in an ice water bath, *p*-toluenesulfonic acid monohydrate (787mg) was added thereto, and the mixture was stirred for about 1.5 hours at room temperature. *t*-Butyl methyl ether (100 mL) was added to the reaction liquid, and the mixture was washed sequentially with a 5% aqueous solution of sodium hydrogen carbonate and water. The organic layer was dried over anhydrous sodium sulfate, followed by filtration, and then the solvent was distilled off under reduced pressure, thereby to obtain 15.24 g (content 13.4 g, yield: quantitative) of the title compound as a red oily matter.
¹H-NMR (400MHz, CDCl₃) δ: 1.53-1.69 (m, 4H), 1.74-1.79 (m, 1H), 1.84-1.88 (m, 1H), 3.53-3.57 (m, 1H), 3.85-3.92 (m, 1H), 3.90 (s, 6H), 4.49 (d, *J*=12.4Hz, 1H), 4.69 (t, *J*=3.6Hz, 1H), 4.75 (d, *J*=12.4Hz, 1H), 6.60 (s, 2H).

### Synthesis of {2,6-dimethoxy-4-[(tetrahydro-2H-pyran-2-yloxy)methyl]pheny 1}boronic acid

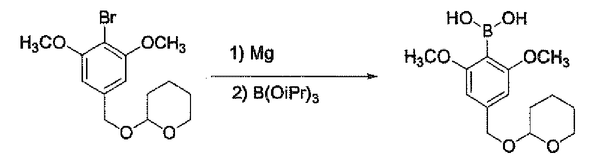

Magnesium (353 mg), tetrahydrofuran (10 mL) and iodine (catalytic amount) were mixed. To this mixture, about a one-third amount of a solution of 2-[(4-bromo-3,5-dimethoxybenzyl)oxy]tetrahydro-2*H*-pyran (5.00 g) in tetrahydrofuran (5.0 mL) was added, and the mixture was heated to 70°C. After it was confirmed that the color of the solution turned clear and colorless, and that the solution began to generate bubbles, the remaining portion (2/3) of the solution of 2-[(4-bromo-3,5-dimethoxybenzyl)oxy]tetrahydro-2*H*-pyran in tetrahydrofuran was injected therein over 11 minutes. The reaction mixture was stirred for 2 hours at 70°C, was subsequently cooled to room temperature, and was diluted with tetrahydrofuran (15 mL). The reaction liquid was cooled in an ice water bath, and triisopropyl borate (3.7 mL) was added dropwise thereto over 6 minutes at -1.1 to -0.1°C. The reaction mixture was stirred for about 2.5 hours at room temperature, and then was cooled in an ice water bath. A 10% aqueous solution of ammonium chloride (50 mL) was added to the reaction mixture, and the mixture was returned to room temperature. Ethyl acetate (50 mL) was added to the reaction mixture to perform partition, and the organic layer was washed with water (organic layer (1), aqueous layer (1)). The target product was extracted again from the aqueous layer (1) with ethyl acetate (25 mL) (organic layer (2)). The organic layer (1) and the organic layer (2) were combined, and the solvent was distilled off under reduced pressure. The concentration residue was purified by silica gel column chromatography, to obtain 2.80 g (content 2.67 g, yield 67.7%) of the title compound as a pale yellow oily matter.
¹H-NMR (400MHz, DMSO-*d*₆) δ: 1.47-1.54 (m, 4H), 1.65-1.74 (m, 2H), 3.46-3.49 (m, 1H), 3.69 (s, 6H), 3.78-3.83 (m, 1H), 4.43 (d, *J*=12.0Hz, 1H), 4.62 (d, *J*=12.0Hz, 1H), 4.65 (t, *J*=3.2Hz, 1H), 6.53 (s, 2H), 7.80 (s, 2H).

### Example C-2:

### Synthesis of N-(cyclopropylmethyl)-7-{2,6-dimethoxy-4-[(tetrahydro-2H-py ran-2-yloxy)methyl]phenyl}-2-ethyl-N-(tetrahydro-2H-pyran-4 -ylmethyl)pyrazolo[1,5-a]pyridin-3-amine

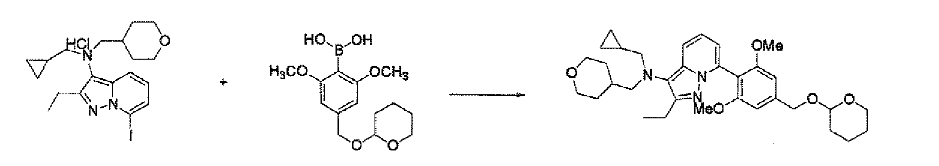

1,2-Dimethoxyethane (17 mL), *N*-cyclopropylmethyl-2-ethyl-7-iodo-*N*-(tetrahydro-2*H*-pyran-4 -ylmethyl)pyrazolo[1,5-*a*]pyridin-3-amine hydrochloride (500 mg), potassium carbonate (507 mg), triphenylphosphine (56 mg), palladium acetate (12.4 mg) and water (8.5 mL) were added sequentially to {2,6-dimethoxy-4-[(tetrahydro-2*H*-pyran-2-yloxy)methyl]pheny 1}boronic acid (530 mg). This mixture was heated to 95°C, and was stirred for about 4 hours. A solution of {2,6-dimethoxy-4-[(tetrahydro-2*H*-pyran-2-yloxy)methyl]pheny 1}boronic acid (132 mg) in 1,2-dimethoxyethane (2.0 mL) was added to the reaction mixture, and the mixture was further stirred for about 2 hours at 95°C. Subsequently, the mixture was cooled to room temperature. Ethyl acetate (20 mL) was added to the reaction mixture to perform partition, and the organic layer was washed sequentially with a 1 N aqueous solution of sodium hydroxide and water (twice). The solvent was distilled off under reduced pressure, to obtain 887 mg (content 592 mg, yield: quantitative) of the title compound as a brown oily matter. ¹H-NMR (400MHz, CDCl₃) δ:-0.02-0.04 (m, 2H), 0.34-0.39 (m, 2H), 0.80-0.90 (m, 1H), 1.23 (t, *J*=7.6Hz, 3H), 1.26-1.32 (m, 2H), 1.54-1.93 (m, 9H), 2. 78 (q, *J*=7.6Hz, 2H), 2.89 (d, *J*=6.4Hz, 2H), 3.06 (d, *J*=6.8Hz, 2H), 3.32 (dt, *J*=1.6, 11.6Hz, 2H), 3.55-3.63 (m, 1H), 3.73 (s, 6H), 3.91-3.99 (m, 3H), 4.59 (d, *J*=12.0Hz, 1H), 4.72 (t, *J*=3.6Hz, 1H), 4.86 (d, *J*=12.4Hz, 1H), 6.60 (dd, *J*=1.2, 6.8Hz, 1H), 6.72 (s, 2H), 7.03 (dd, *J*=6.8, 8.8Hz, 1H), 7.46 (dd, *J*=1.2, 8.8Hz, 1H).

### Example C-3:

### Synthesis of (4-{3-[(cyclopropylmethyl)(tetrahydro-2H-pyran-4-ylmethyl)a mino]-2-ethylpyrazolo[1,5-a]pyridin-7-yl}-3,5-dimethoxyphen yl)methanol

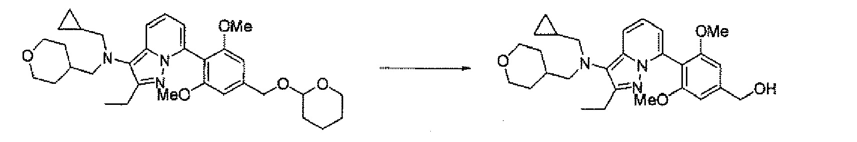

Toluene (5.7 mL) and 5 N hydrochloric acid (5.7 mL) were added sequentially to *N-(-*cyclopropylmethyl)-7-{2,6-dimethoxy-4-[(tetrahydro-2*H*-py ran-2-yloxy)methyl]phenyl)-2-ethyl-*N*-(tetrahydro-2*H*-pyran-4 -ylmethyl)pyrazolo[1,5-*a*]pyridin-3-amine (858 mg), and the mixture was stirred for about 3 hours at room temperature. Toluene (2 mL) was added to the reaction mixture to perform partition. Ethyl acetate (11 mL) was added to the aqueous layer, and the mixture was stirred under ice cooling. A 5 N aqueous solution of sodium hydroxide (6.0 mL) was added dropwise thereto, and the aqueous layer was adjusted to be strongly alkaline (pH 13). Ethyl acetate (2 mL) was added to this mixture to perform partition, and the organic layer was washed with water. The solvent was distilled off under reduced pressure, to obtain 461 mg (content 405 mg, yield 82.8%) of the title compound as a yellow oily matter. ¹H-NMR (400MHz, CDCl₃) δ_{:} -0.02-0.04 (m, 2H), 0.35-0.39 (m, 2H), 0.80-0.90 (m, 1H) 1.23 (t, *J*=7.6Hz, 3H), 1.24-1.34 (m, 2H), 1.54-1.64 (m, 1H), 1.77 (dd, *J*=2.0, 12. 8Hz, 2H), 2. 36 (t, *J*=5.6Hz, 1H), 2.78(q, *J*=7.6Hz, 2H), 2.89(d, *J*=6.8Hz, 2H), 3.06 (d, *J*=7.2Hz, 2H), 3.32 (dt, *J*=2.0, 11.6Hz, 2H), 3.74 (s, 6H), 3. 96 (dd, *J*=2.8, 11. 6Hz, 2H), 4.71 (d, *J*=6.0Hz, 2H), 6. 61 (dd, *J*=1.6, 6.8Hz, 1H), 6.71(s, 2H), 7.04 (dd, *J*=6.8, 8.8Hz, 1H), 7.47 (dd, *J*=1.6, 8.8Hz, 1H).

### Example D. Synthesis of N-cyclopropylmethyl-7-(2,6-dimethoxy-4-methoxymethylphenyl) -2-ethyl-N-(tetrahydro-2H-pyran-4-ylmethyl)pyrazolo[1,5-a]p yridin-3-amine

### Example D-1:

### Synthesis of N-cyclopropylmethyl-7-(2,6-dimethoxy-4-methoxymethylphenyl) -2-ethyl-N-(tetrahydro-2H-pyran-4-ylmethyl)pyrazolo[1,5-a]p yridin-3-amine

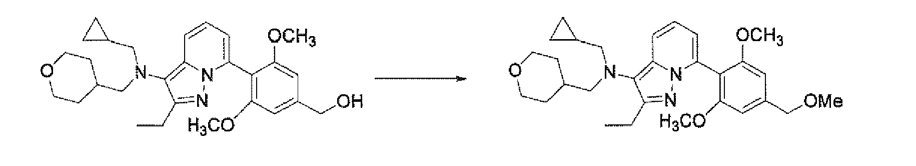

Dimethyl sulfate (0.315 mL) was added to a mixture of (4-{3-[(cyclopropylmethyl)(tetrahydro-2*H*-pyran-4-ylmethyl)a mino]-2-ethylpyrazolo[1,5-*a*]pyridin-7-yl}-3,5-dimethoxyphen yl)methanol (1 g) and tetrahydrofuran (6 mL). This mixed liquid was divided into three parts, and the mixed liquid was added dropwise to a mixture of 60% sodium hydride (133 mg) and tetrahydrofuran (3 mL) at room temperature while the liquid was stirred. After completion of the dropwise addition, the mixture was further stirred for 6 hours and 14 minutes at room temperature, and 1 mL of water was added thereto. Subsequently, 9 mL of isopropyl acetate and 8 mL of water were added to perform partition, and thus an upper layer was obtained. The upper layer was washed with 9 mL of 2% brine, and then was concentrated under reduced pressure at 50°C, to obtain 1.138 g of the target product as a yellowish oil (quantified yield 94.7%).
HPLC: L-column ODS (250 x 4.6 mm), mobile phase: acetonitrile/water/trifluoroacetic acid = 400/600/5 (v/v/v), wavelength: 296 nm, flow rate: 1.0 m/min, analysis temperature: room temperature, retention time: 11.67 minutes.

### Example D-2:

### Synthesis of N-cyclopropylmethyl-7-(2,6-dimethoxy-4-methoxymethylphenyl) -2-ethyl-N-(tetrahydro-2H-pyran-4-ylmethyl)pyrazolo[1,5-a]p yridin-3-amine

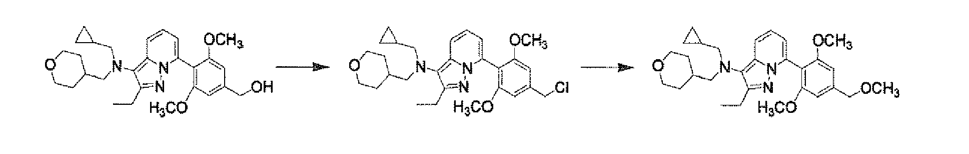

To a mixture of (4-{3-[(cyclopropylmethyl)(tetrahydro-2*H*-pyran-4-ylmethyl)a mino]-2-ethylpyrazolo[1,5-alpyridin-7-yl}-3,5-dimethoxyphen yl) methanol (1 g), toluene (6.7 mL) and 1,2-dimethoxyethane (3.3 mL), thionyl chloride (200 µL) was added under stirring, and the mixture was further stirred for 58 minutes at the same temperature. 28% Sodium methoxide/methanol (4.01 g) was added thereto, and the mixture was warmed and stirred for 7 hours and 15 minutes at 80°C. 10 mL of isopropyl acetate and 10 mL of water were added to perform partition, and thus an upper layer was obtained. The upper layer was washed with 10 mL of 2% brine, and then was concentrated under reduced pressure, to obtain 929.57 mg of the target product as a yellow oil (quantified yield 91.6%).
HPLC: L-column ODS (250 x 4.6 mm), mobile phase: acetonitrile/water/trifluoroacetic acid = 400/600/5 (v/v/v), wavelength: 29 nm, flow rate: 1.0 mL/min, analysis temperature: room temperature, retention time: 11.93 minutes.

### Example D-3:

To a mixture of (4-{3-[(cyclopropylmethyl)(tetrahydro-2*H*-pyran-4-ylmethyl)a mino]-2-ethylpyrazolo[1,5-*a*]pyridin-7-yl}-3,5-dimethoxyphen yl)methanol (9.0 g), toluene (57 mL) and 1,2-dimethoxyethane (28.5 mL), thionyl chloride (1.73 mL) was added under stirring at room temperature under a nitrogen atmosphere (internal temperature 19.9 to 26.9°C). The mixture was further stirred for 44 minutes at room temperature, and 28% sodium methoxide/methanol (34.4 g) was added. The mixture was warmed and stirred for 1 hour and 54 minutes at a bath temperature of 80°C. The mixture was cooled in a water bath, and then 85.5 mL of toluene and 85.5 mL of water were added to perform partition. Thus, an upper layer was obtained. The upper layer was extracted sequentially with 85.5 mL and 28. 5 mL of 5 N hydrochloric acid, and the extracts were combined. 128.3 mL of isopropyl acetate and 115.4 mL of a 5 N aqueous solution of sodium hydroxide were added to the hydrochloric acid extracted layer, and the target product was extracted into the upper layer. The lower layer was disposed, and then an aqueous solution of ethylenediamine (EDA water) was added to the upper layer, and the mixture was stirred vigorously for 36 minutes at room temperature (EDA water = 7.7 mL of EDA + 85.5 mL of water). After the mixture was left to stand still, the lower layer was disposed, and subsequently the same EDA water washing was performed two times (second time: stirring for 30 minutes, third time: stirring for 33 minutes). The resultant was washed two times with 85.5 mL of water, and then was concentrated under reduced pressure at a bath temperature of 50°C. Subsequently, the residue was azeotropically boiled with 60 mL of ethanol, to obtain 8.447 g of the target product (quantified yield 97.4%).
HPLC: L-column ODS (250 x 4.6 mm), mobile phase: acetonitrile/water/perchloric acid = 500/500/5 (v/v/v), wavelength: 296 nm, flow rate: 1. 0 mL/min, analysis temperature: room temperature, retention time: 8.37 minutes.

### Example D-4:

### Synthesis of N-cyclopropylmethyl-7-(2,6-dimethoxy-4-methoxymethylphenyl) -2-ethyl-N-(tetrahydro-2H-pyran-4-ylmethyl)pyrazolo[1,5-a]p yridin-3-amine

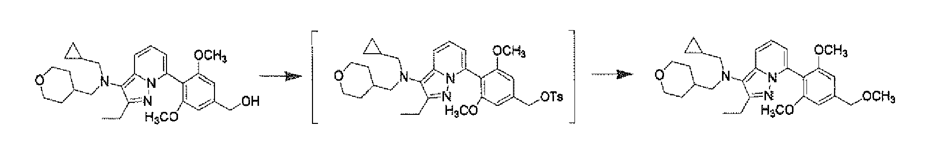

To a mixture of (4-(3-[(cyclopropylmethyl)(tetrahydro-2H-pyran-4-ylmethyl)a mino]-2-ethylpyrazolo[1,5-*a*]pyridin-7-yl}-3,5-dimethoxyphen yl)methanol (700 mg), toluene (7 mL), triethylamine (0.47 mL), 1,2-dimethoxyethane (3.5 mL) and N-methylimidazole (27.6 mg), *p*-toluenesulfonyl chloride (614 mg) was added at 0°C, and the mixture was stirred for 4.5 hours. Sodium methoxide (28% methanol solution) (10.5 mL) was added to this mixture, and the mixture was stirred for about 2 hours at room temperature. Water (7 mL) was added to the mixture, and then toluene (7 mL) was added. The mixture was sufficiently shaken, and then the organic layer was collected by separation. The mixture was filtered to obtain a filtrate, and the solvent was distilled off under reduced pressure. Toluene (7 mL) and water (4 mL) was added to the residue. The mixture was sufficiently shaken and then the organic layer was collected by separation. The mixture was filtered to obtain a filtrate, and the solvent was distilled off under reduced pressure. The resulting residue was purified by column chromatography (20 to 30% ethyl acetate/heptane), to obtain 686.2 mg (yield 95.2%) of the title compound.
HPLC: L-Column ODS (250 x 4.6 mm), mobile phase: acetonitrile/water/trifluoroacetic acid = 500/500/5 (v/v/v), wavelength: 296 nm, flow rate: 1.0 mL/min, analysis temperature: room temperature, retention time: 5.99 minutes.

### Example E: Synthesis of 2-[2,6-dimethoxy-4-(methoxymethyl)phenyl]-5,5-dimethyl-1,3, 2-dioxaborinane and N-cyclopropylmethyl-7-(2,6-dimethoxy-4-methoxymethylphenyl) -2-ethyl-N-(tetrahydro-2H-pyran-4-ylmethyl)pyrazolo[1,5-a]p yridin-3-amine

### Example E-1-1:

### Synthesis of 2-[2,6-dimethoxy-4-(methoxymethyl)phenyl]-5,5-dimethyl-1,3, 2-dioxaborinane

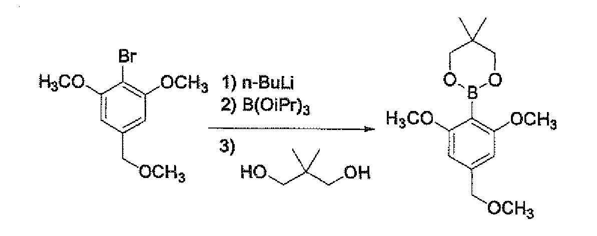

1-Bromo-2,6-dimethoxy-4-methoxymethylbenzene (0.78 g) was dissolved in tetrahydrofuran (4 mL) under a nitrogen atmosphere. A 2.77 M *n*-butyllithium-hexane solution (1.13 mL) was added dropwise thereto at an internal temperature of -68.5 to -54.6°C, and the mixture was stirred for one hour at the same temperature (reaction liquid A). In addition, tetrahydrofuran (4 mL) was introduced into triisopropyl borate (1.04 mL) under a nitrogen atmosphere, and the mixture was stirred while being cooled in a dry ice-acetone bath (reaction liquid B). The reaction liquid A was added dropwise to the reaction liquid B at an internal temperature of -72.8 to -64.4°C, and the mixture was stirred for one hour at the same temperature. Thereafter, the mixture was stirred for 33 minutes at room temperature, and acetic acid (0.1 mL) and 2,2-dimethyl-1, 3-propanediol (195 mg) were added thereto. The mixture was stirred for one hour at room temperature. *t*-Butyl methyl ether (10 mL) and a saturated aqueous solution of ammonium chloride (10 mL) were added to perform partition, and an upper layer was obtained (waste water layer 1). The upper layer was washed sequentially with 10 mL of water (waste water layer 2) and 10 mL of saturated brine (waste water layer 3) (organic layer 1). Subsequently, *t*-butyl methyl ether (10 mL) was added to the waste water layers 1+2, and the mixture was partitioned. The organic layer was washed with the waste water layer 3 (organic layer 2). The organic layers 1+2 were combined, and the mixture was dried over anhydrous sodium sulfate and concentrated under reduced pressure at 40°C. The concentration residue was purified by silica gel chromatography (t-butyl methyl ether/heptane = 1/1 to 2/1), to obtain 601 mg of the target product (yield 68.3%).
¹*H*-NMR (CDCl₃): δ: 1.1 (s, 6H), 3.35 (s, 3H), 3.79 (s, 6H), 3.79 (s, 4H), 4.43 (s, 2H), 6.47 (s, 2H).

### Example E-1-2:_

### Synthesis of 2-[2,6-dimethoxy-4-(methoxymethyl)phenyl]-5,5-dimethyl-1,3, 2-dioxaborinane

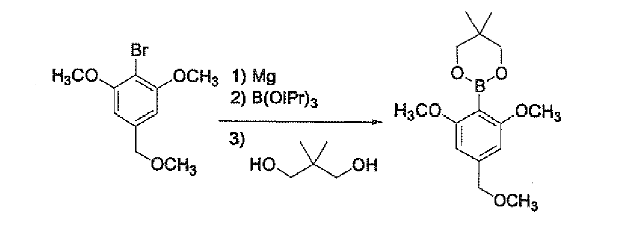

Tetrahydrofuran (19 mL) was added to magnesium (0.29 g), and the mixture was stirred at 80°C under a nitrogen atmosphere. 1 mL of a solution prepared by dissolving 1-bromo-2,6-dimethoxy-4-methoxymethylbenzene (3 g) in tetrahydrofuran (3 mL) was introduced thereto under heating, and subsequently a solution prepared by dissolving iodine (14 mg) in tetrahydrofuran (0.5 mL) was introduced thereto. The remaining 1-bromo-2,6-dimethoxy-4-methoxymethylbenzene/tetrahydrofura n solution was introduced thereto, and then the mixture was refluxed for 61 minutes at a bath temperature of 80°C. The mixture was left to cool naturally at room temperature, and then triisopropyl borate (3.1 mL) was added thereto in an ice bath at an internal temperature of -0.4 to 4.0°C. The mixture was stirred again for 63 minutes at room temperature, and then acetic acid (0.77 mL) and 2,2-dimethyl-1,3-propanediol (1.54 g) were added thereto under ice cooling. The mixture was stirred for 18.1 hours at room temperature, and then an aqueous solution (22.5 mL) containing 5% sodium hydrogen carbonate and 5% sodium chloride dissolved therein was added thereto in an ice bath. The mixture was stirred for 5 minutes. After the mixture was left to stand, an organic layer was obtained by performing partition. The lower layer was re-extracted with ethyl acetate (12 mL), and was combined with the previously obtained organic layer. The combined extracted layer was dried over anhydrous magnesium sulfate, was then concentrated under reduced pressure, and was further purged with 6 mL of toluene. 6 mL of toluene was added to the concentration residue, and the mixture was heated to dissolve in a bath at 50°C. When the mixture was left to cool naturally at room temperature, a small amount of solid was precipitated. Therefore, after seed crystals were introduced, the mixture was stirred for 5 minutes at room temperature. 9 mL of heptane was added, and the mixture was stirred for 80 minutes in an ice bath. Subsequently, the precipitates were collected by filtration and were washed with heptane (6 mL). The precipitates were dried in a vacuum at room temperature, to obtain 2.625 g of the target product (yield 77.6%).
¹H-NMR (CDCl₃): δ: 1.10 (s, 6H), 3.34 (s, 3H), 3.80 (s, 6H), 3.80 (s, 4H), 4.40 (s, 2H), 6.46 (s, 2H).

### Example E-2:

### Synthesis of N-cyclopropylmethyl-7-(2,6-dimethoxy-4-methoxymethylphenyl) -2-ethyl-N-(tetrahydro-2H-pyran-4-ylmethyl)pyrazolo[1,5-a]p yridin-3-amine

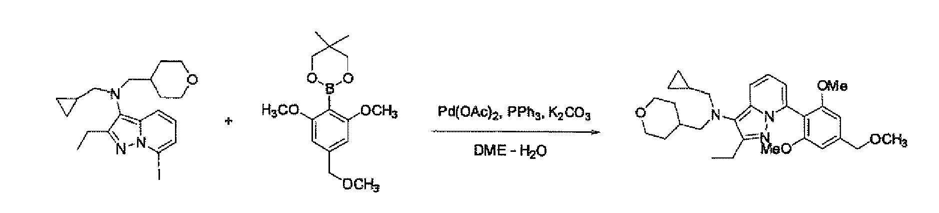

N-(cyclopropylmethyl)-2-ethyl-7-iodo-N-(tetrahydro-2 *H*-pyran-4-ylmethyl)pyrazolo[1,5-a]pyridin-3-amine (215 mg), palladium acetate (13 mg), triphenylphosphine (51 mg) and potassium carbonate (171 mg) were added to 2-[2,6-dimethoxy-4-(methoxymethyl)phenyl]-5,5-dimethyl-1,3, 2-dioxaborinane (230 mg), and the system was purged with nitrogen. 1,2-Dimethoxyethane (4.31mL) and water (1.08mL) were introduced thereto, and the mixture was heated under stirring for 14.6 hours at a bath temperature of 90°C. Some of the reaction liquid was sampled and analyzed by HPLC, and it was found that the target product was produced at a proportion of 92.11% by area percentage.
L-column ODS, 4. 6 x 250 mm, wavelength: 296 nm, flow rate: 1.0 mL/min, column temperature: room temperature, mobile phase: acetonitrile/water/trifluoroacetic acid = 400/600/5 (v/v/v), retention time: 11.97 minutes.

### F. Synthesis of potassium

### [2,6-dimethoxy-4-(methoxymethyl)phenyl]trifluoroboronate and N-cyclopropylmethyl-7-(2,6-dimethoxy-4-methoxymethylphenyl) -2-ethyl-N-(tetrahydro-2H-pyran-4-ylmethyl)pyrazolo[1,5-a]p yridin-3-amine

### Example F-1:

### Synthesis of potassium [2,6-dimethoxy-4-(methoxymethyl)phenyl]trifluoroboronate

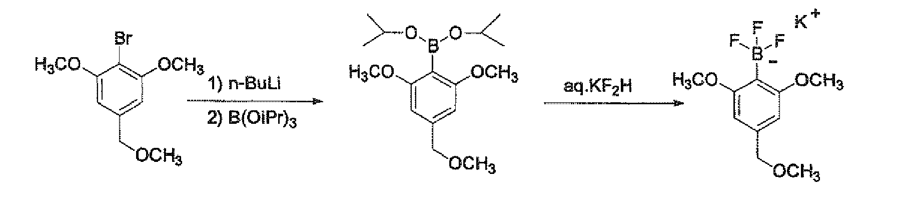

1-Bromo-2,6-dimethoxy-4-methoxymethylbenzene (1 g) was dissolved in tetrahydrofuran (7.5 mL) under a nitrogen atmosphere. A 2.77 M n-butyllithium-hexane solution (1.45 mL) was added dropwise thereto at an internal temperature of -75.1 to -63.1°C, and the mixture was stirred for one hour at an internal temperature of -76.1 to -70.0°C. Triisopropyl borate (1 mL) was added dropwise thereto at an internal temperature of -72.7 to -62.1°C, and subsequently the mixture was stirred for 56 minutes at an internal temperature of -77.5 to -68.3°C. The mixture was stirred for 1.3 hours at room temperature, and then a liquid formed by dissolving potassium hydrogen fluoride (0.9 g) in 5 mL of water was added dropwise thereto at an internal temperature of 5°C or below. Subsequently, the mixture was stirred for 4.3 hours at room temperature. Precipitated solids were collected by filtration and were washed with methanol/water = 1/1 (5 mL). The resulting solid was dried in a vacuum for several minutes, to obtain 480 mg of the target product (yield 43.5%). ¹H-NMR (DMSO): δ: 3.26 (s, 3H), 3.56 (s, 6H), 3.91 (s, 2H), 6.33 (s, 2H).

### Example F-2:

### Synthesis of

### N-cyclopropylmethyl-7-(2,6-dimethoxy-4-methoxymethylphenyl) -2-ethyl-N-(tetrahydro-2H-pyran-4-ylmethyl)pyrazolo[1,5-a]p yridin-3-amine

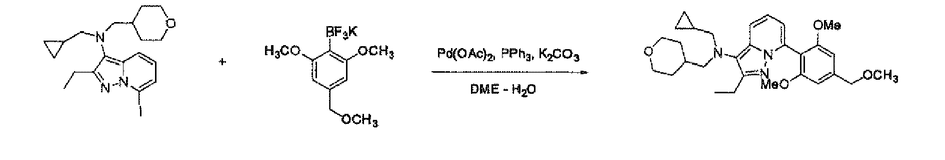

*N*-(cyclopropylmethyl)-2-ethyl-7-iodo-*N*(tetrahydro-2 *H*-pyran-4-ylmethyl)pyrazalo[1,5-*a*]pyridin-3-amine (216 mg), palladium acetate (13.1 mg), triphenylphosphine (51.2 mg) and potassium carbonate (172 mg) were added to potassium [2,6-dimethoxy-4-(methoxymethyl)phenyl]trifluoroboronate (224 mg), and the system was purged with nitrogen. 1, 2-Dimethoxyethane (7.2 mL) and water (3. 6 mL) were introduced thereto, and the mixture was heated under stirring for 18.5 hours at a bath temperature of 90°C. Some of the reaction liquid was sampled and analyzed by HPLC, and it was found that the target product was produced at a proportion of 92.49% by area percentage.

L-column ODS, 4. 6 x 250 mm; wavelength: 296 nm; flow rate: 1.0 mL/min. Column temperature: room temperature, mobile phase: acetonitrile/water/trifluoroacetic acid 400/600/5 (v/v/v).

Retention time: 11.91 minutes.

## Claims

1. A method for producing a compound (I) represented by following formula, or a salt thereof, comprising:
a step A of converting a hydroxyl group in a compound (IV) represented by following formula into a methoxy group, thereby to obtain the compound (I) or the salt thereof:

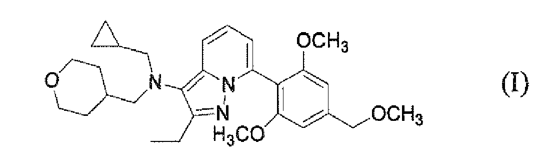

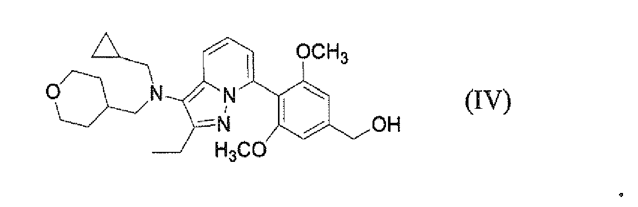

2. The method according to claim 1, wherein the step A is carried out by using a methylating agent.

3. The method according to claim 1, wherein the step A comprises a step A-1 of converting a hydroxyl group of the compound (IV) into a leaving group, and a step A-2 of substituting the leaving group with a methoxy group.

4. The method according to claim 3, wherein the step A-1 is carried out by using at least one selected from the group consisting of sulfonyl chloride, thionyl halide and phosphorus halide.

5. The method according to claim 3, wherein the step A-2 is carried out by using a combination of an alkali metal hydroxide and methanol, or a metal alkoxide.

6. The method according to any one of claims 1 to 5, further comprising, before the step A, a step B of reacting a compound (II) represented by the following formula or a salt thereof with a compound (III') represented by the following formula, and converting, in a case where R¹ is a protective group P¹, -OR¹ in the resulting compound into a hydroxyl group, to obtain the compound (IV): 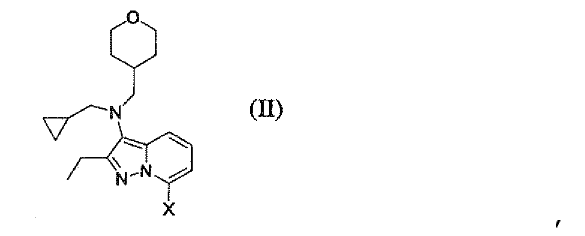 wherein X represents a halogen; 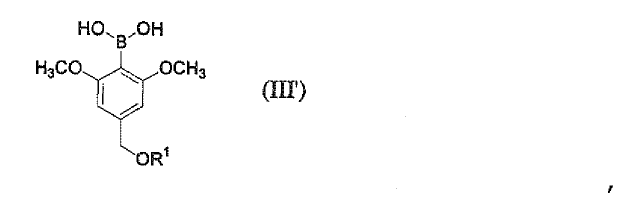 wherein R¹ represents a hydrogen atom or a protective group P¹; P¹ represents a group selected from the group consisting of a methyl group which may have substituents, an ethyl group which may have substituents, a benzyl group which may have substituents and a silyl group.

7. The method according to claim 6, comprising, before the step B, a step of removing the protective group P¹ of a compound (III") represented by the following formula, to obtain a compound (III) represented by the following formula as the compound (III'): 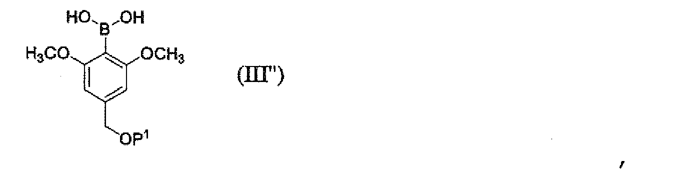 wherein the protective group P¹ has the same definition as described above;

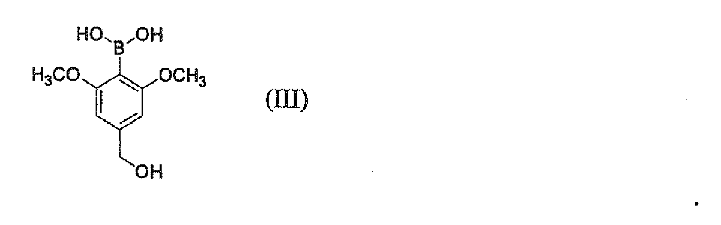

8. A method for producing a compound (I) or a salt thereof, comprising a step of reacting a compound (II) represented by the following formula or a salt thereof, with a compound (V) represented by the following formula or a compound (VI) represented by the following formula, to obtain the compound (I) represented by the following formula or a salt thereof: 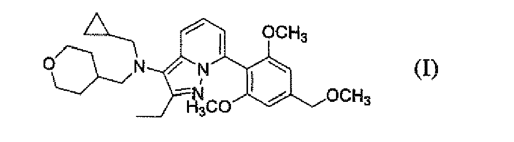 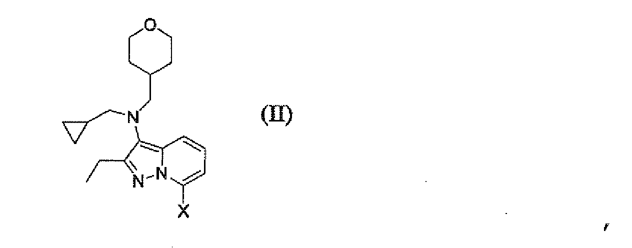 wherein X represents a halogen; 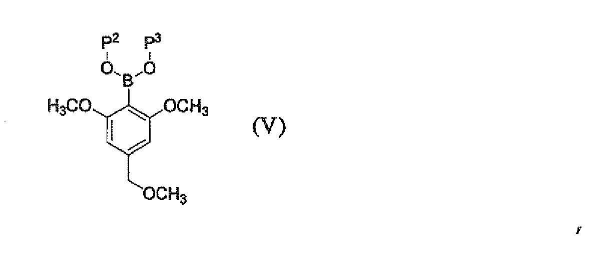 wherein P² and P³ are joined together with -O-B-O- to represent a group represented by any one of the following formulae A-1 to A-4: 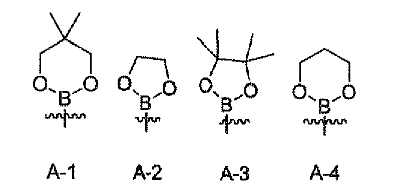 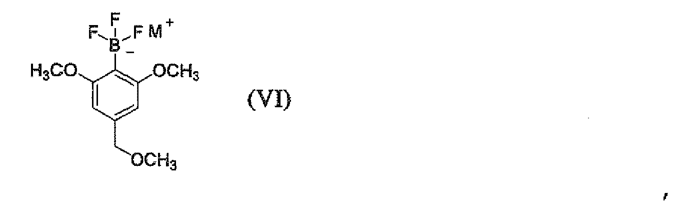 wherein M represents an alkali metal, an alkaline earth metal or an ammonium.

9. A compound (IV') represented by the following formula or a salt thereof: 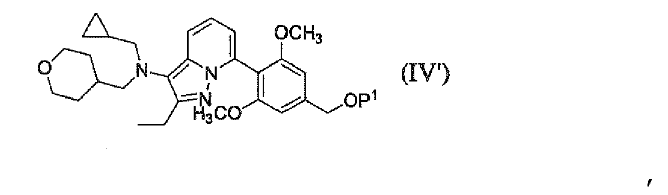 wherein P¹ represents a group selected from the group consisting of a methyl group which may have substituents, an ethyl group which may have substituents, a benzyl group which may have substituents and a silyl group.

10. A compound (III") represented by the following formula, a compound (V) represented by the following formula, a compound (VI) represented by the following formula, or a salt thereof: 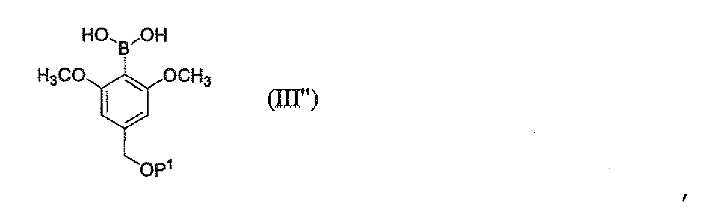 wherein P¹ represents a group selected from the group consisting of a methyl group which may have substituents, an ethyl group which may have substituents, a benzyl group which may have substituents, and a silyl group; 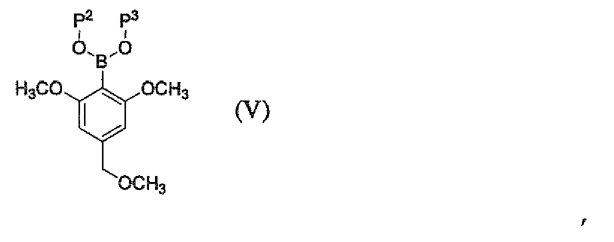 wherein P² and P³ are joined together with -O-B-O- to represent a group represented by any one of the following formulae A-1 to A-4; 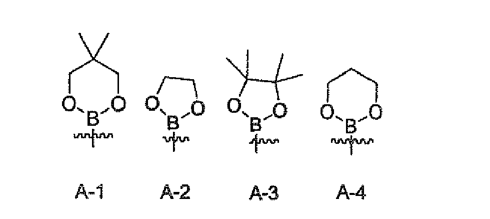 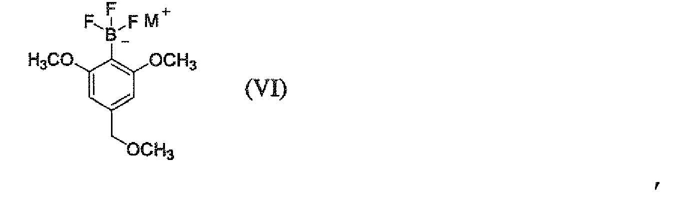 wherein M represents an alkali metal, an alkaline earth metal or an ammonium.
